(19) Europäisches Patentamt
European Patent Office
Office européen des brevets

(11) EP 4 592 246 A1

(12) **EUROPEAN PATENT APPLICATION**
published in accordance with Art. 153(4) EPC

(43) Date of publication:
**30.07.2025 Bulletin 2025/31**

(21) Application number: **23867497.2**

(22) Date of filing: **19.09.2023**

(51) International Patent Classification (IPC):
***C01B 37/02*** *(2006.01)*

(52) Cooperative Patent Classification (CPC):
**B01J 29/03; B01J 29/035; C01B 37/02; C07D 201/04; C07D 223/10**

(86) International application number:
**PCT/CN2023/119697**

(87) International publication number:
**WO 2024/061210 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.09.2022 CN 202211139853**

(71) Applicants:
- **China Petroleum & Chemical Corporation**
  **Beijing 100728 (CN)**
- **Sinopec Research Institute of Petroleum Processing Co., Ltd.**
  **Beijing 100083 (CN)**

(72) Inventors:
- **XIA, Changjiu**
  **Beijing 100083 (CN)**
- **ZHANG, Peng**
  **Beijing 100083 (CN)**
- **PENG, Xinxin**
  **Beijing 100083 (CN)**
- **XING, Enhui**
  **Beijing 100083 (CN)**
- **ZHANG, Xiaoxin**
  **Beijing 100083 (CN)**
- **LUO, Yibin**
  **Beijing 100083 (CN)**
- **LIN, Min**
  **Beijing 100083 (CN)**
- **ZHU, Bin**
  **Beijing 100083 (CN)**
- **SHU, Xingtian**
  **Beijing 100083 (CN)**

(74) Representative: **Hoffmann Eitle**
**Patent- und Rechtsanwälte PartmbB**
**Arabellastraße 30**
**81925 München (DE)**

# (54) HIERARCHICAL PORE ALL-SILICON MOLECULAR SIEVE, PREPARATION METHOD THEREFOR, AND USE THEREOF

(57) Disclosed is a hierarchical porous all-silica molecular sieve, which is characterized by solid nuclear magnetic resonance technology using trimethylphosphine (TMP) as a probe molecule. The $^{31}P$ MAS NMR spectrum of the molecular sieve shows three characteristic peaks at chemical shifts of -5±1 ppm, -34±1 ppm and -61±1 ppm, wherein the peak intensity of the characteristic peak at the chemical shift of -5±1 ppm is recorded as $N_1$, the peak intensity of the characteristic peak at the chemical shift of - 34±1 ppm is recorded as $N_2$, and the peak intensity of the characteristic peak at the chemical shift of -61±1 ppm is recorded as $N_3$, then $X_1$ as defined by formula (1) is in the range of 0.10-0.30; $X_1 = N_1 / N_3$ (1); and $X_2$ as defined by formula (2) is in the range of 0.15-0.45; $X_2 = N_2 / N_3$ (2). When the molecular sieve is used in the gas-phase Beckmann rearrangement reaction of cyclohexanone oxime, the selectivity of the caprolactam product and the catalyst life can be improved.

EP 4 592 246 A1

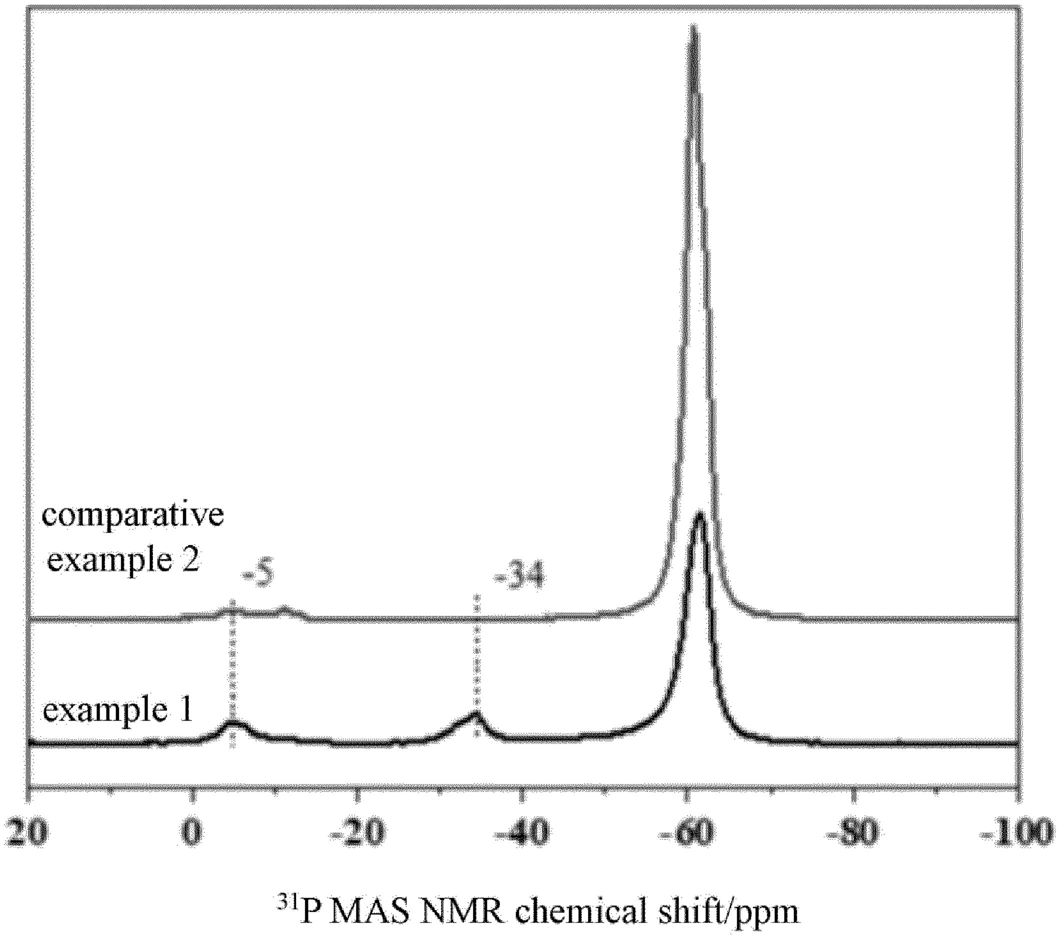
comparative
example 2
-5
-34
example 1
20
0
-20
-40
-60
-80
-100
$^{31}$P MAS NMR chemical shift/ppm

FIG. 1

**Description**

Technical Field

The present application relates to the technical field of all-silica molecular sieves, and in particular to a hierarchical porous all-silica molecular sieve and a preparation method and application thereof.

Background Art

ε-caprolactam (ε-CPL) is an important monomer for the synthesis of nylon-6. It is widely used in the production of important downstream products such as engineering plastics, nylon-6 fibers and industrial cord fabrics. It can also be used in coatings, pharmaceuticals and fine chemicals. At present, more than 90% of CPL is prepared by liquid-phase Beckmann rearrangement reaction using cyclohexanone oxime (CHO), wherein concentrated sulfuric acid (or fuming sulfuric acid) is used as catalyst and solvent. There are serious problems such as equipment corrosion and environmental pollution. After the reaction, liquid ammonia is used to neutralize the waste sulfuric acid, resulting in a large amount of low-value ammonium sulfate by-product (1.9 t ammonium sulfate/t CPL), resulting in poor technical and economic performance of this route.

Therefore, the CHO gas-phase Beckmann rearrangement reaction process based on heterogeneous catalysts has received great attention from academia and industry. Compared with the traditional liquid phase method, this process avoids the use of ammonia and fuming sulfuric acid from the source, with an atomic utilization rate of 100%, and is an environmental friendly CPL green production process. At present, a variety of solid catalysts including molecular sieves, metal oxides and mesoporous materials have been applied to the study of CHO gas-phase Beckmann rearrangement reaction, among which Silicalite-1 all-silica molecular sieve with MFI topological structure has shown excellent catalytic performance. Japan Sumitomo and Sinopec have successively used Silicalite-1 all-silica molecular sieve catalysts to carry out industrial demonstration tests of CHO gas-phase Beckmann rearrangement.

However, CHO gas-phase Beckmann rearrangement route has two problems: poor product CPL selectivity and short catalyst single-pass life, which affect the technical economy and operational continuity and stability, resulting in extremely slow subsequent large-scale industrial expansion and commercial promotion. This is because the micropore size of the MFI structure is small, close to the molecular size of the reactants and products, which leads to slow diffusion of guest molecules in the confined pores of the molecular sieve and significantly prolonged residence time in the crystal, thus aggravating the generation of by-products and the clogging of the pores by carbon deposits.

Ferdi Schüth's research group added 1,7-dichlorooctamethyltetrasilylating agent to form pores during the crystallization process of the molecular sieve and synthesized a hierarchical porous silicalite-1 all-silica molecular sieve (Li W C, Lu A H, Palkovits R, et al. Hierarchically structured monolithic silicalite-1 consisting of crystallized nanoparticles and its performance in the Beckmann rearrangement of cyclohexanone oxime[J]. Journal of the American Chemical Society, 2005, 127(36): 12595-12600). Chao Ge et al. synthesized a hierarchical porous silicalite-1 all-silica molecular sieve with orthogonal stacking of layers by adjusting the length of the alkyl chain in the diquaternary ammonium salt templating agent (Ge, C.; Sun, X. J.; Lian, D. D.; Li, Z. K.; Wu, J. B. Controllable synthesis and structure-performance relationship of silicalite-1 nanosheets in vapor phase beckmann rearrangement of cyclohexanone oxime. Catal. Lett. 2020, 151, 1488-1498).

However, when the hierarchical porous all-silica molecular sieve synthesized by the above technology is used in the gas-phase Beckmann rearrangement reaction of cyclohexanone oxime, the effect of improving caprolactam selectivity and catalyst life is not ideal, and there is still a certain gap from the requirements of industrial production.

Summary of the Invention

The purpose of the present application is to provide a hierarchical porous all-silica molecular sieve and a preparation method and application thereof. When the molecular sieve is used in the gas-phase Beckmann rearrangement reaction of cyclohexanone oxime, the caprolactam selectivity and catalyst life can be improved.

In order to achieve the above object, in one aspect, the present application provides a hierarchical porous all-silica molecular sieve, which is characterized by solid nuclear magnetic resonance technology using trimethylphosphine (TMP) as a probe molecule, and the $^{31}P$ MAS NMR spectrum of the molecular sieve (also referred to as $^{31}P$-TMP MAS NMR spectrum) shows three characteristic peaks at chemical shifts of -5±1 ppm, -34±1 ppm and -61±1 ppm, wherein the peak intensity of the characteristic peak at the chemical shift of -5±1 ppm is recorded as $N_1$, the peak intensity of the characteristic peak at the chemical shift of -34±1 ppm is recorded as $N_2$, and the peak intensity of the characteristic peak at the chemical shift of -61±1 ppm is recorded as $N_3$, then:

$X_1$ as defined by the following formula (1) is in the range of 0.10-0.30, preferably in the range of 0. 15-0.25:

$$X_1 = N_1 / N_3 \ (1);$$

and

$X_2$ as defined by the following formula (2) is in the range of 0.15-0.45, preferably in the range of 0.2-0.35:

$$X_2 = N_2 / N_3 \ (2).$$

In another aspect, a method for preparing the hierarchical porous all-silica molecular sieve of the present application is provided, comprising the following steps:

1) mixing a silicon source, a first templating agent, water, a silanizing agent and a structural filler to obtain a reaction mixture, wherein the structural filler is selected from an amphiphilic surfactant, a hard templating agent or a combination thereof, and the hard templating agent is selected from a natural or synthetic polymer material;
2) sequentially performing a first hydrothermal crystallization treatment and a first calcination treatment on the reaction mixture to obtain a molecular sieve intermediate;
3) mixing the molecular sieve intermediate, a second templating agent and water, and then sequentially performing a second hydrothermal crystallization treatment and a second calcination treatment.

Preferably, the first templating agent and the second templating agent are organic bases, and are independently selected from quaternary ammonium bases, aliphatic amines, aliphatic alcohol amines, or a combination thereof.

In another aspect, a method for preparing caprolactam from cyclohexanone oxime is provided, comprising the step of subjecting cyclohexanone oxime to a gas-phase Beckmann rearrangement reaction in the presence of the hierarchical porous all-silica molecular sieve of the present application.

The hierarchical porous all-silica molecular sieve of the present application has abundant silanol group active centers and has both Brönsted acid and Lewis acid properties, and the value of $X_1$ is in the range of 0.10-0.30, and the value of $X_2$ is in the range of 0.15-0.45, so that the molecular sieve has suitable acidity and has a synergistic catalytic effect on the gas-phase Beckmann rearrangement reaction of cyclohexanone oxime, thereby significantly improving the caprolactam selectivity and extending the catalyst life.

Other features and advantages of the present application will be described in detail in the subsequent Specific Mode for Carrying out the Invention section.

Description of Drawings

The drawings are used to provide a further understanding of the present application and constitute a part of the specification. Together with the following specific embodiments, they are used to explain the present application but do not constitute a limitation to the present application. In the drawings:

FIG 1 is a $^{31}$P-TMP MAS NMR spectrum of the molecular sieve product obtained in Example 1 and the molecular sieve product obtained in Comparative Example 2;
FIG 2 is an XRD spectrum of the molecular sieve product obtained in Example 1;
FIG 3 is a TEM electron microscope image of the molecular sieve product obtained in Example 1;
FIG 4 is a SEM electron microscope image of the molecular sieve product obtained in Example 1;
FIG 5 is a BET curve of the molecular sieve product obtained in Example 1;
FIG 6 is an infrared hydroxyl spectrum of the molecular sieve product obtained in Example 1;
FIG 7 is a TEM electron microscope image of the intermediate obtained in Example 1;
FIG 8 is an XRD spectrum of the molecular sieve product obtained in Example 9;
FIG 9 is an XRD spectrum of the molecular sieve product obtained in Example 10;
FIG 10 is a TEM electron microscope image of the molecular sieve product obtained in Comparative Example 2.

Specific Mode for Carrying out the Invention

The specific implementation of the present application is described in detail below. It should be understood that the specific implementation described here is only used to illustrate and explain the present application, and is not used to limit the present application.

Any specific numerical value disclosed herein (including the endpoint of the numerical range) is not limited to the exact value of the numerical value, but should be understood to also cover values close to the exact value, such as all possible values within the range of ±5% of the exact value. In addition, for the disclosed numerical range, the endpoint

values of the range, the endpoint values and the specific point values in the range, and the specific point values can be arbitrarily combined to obtain one or more new numerical ranges, and these new numerical ranges should also be regarded as specifically disclosed herein.

Unless otherwise specified, the terms used herein have the same meaning as commonly understood by those skilled in the art. If a term is defined herein and its definition is different from the commonly understood meaning in the art, the definition herein shall prevail.

In the present application, the term "hierarchical porous" has the meaning generally understood in the art, specifically referring to a molecular sieve having multiple levels of pore sizes, for example, two levels of pore sizes of micropores (pore size <2 nm) and mesopores (pore size of 2-50 nm), micropores and macropores (pore size > 50 nm), or mesopores and macropores, or three levels of pore sizes of micropores, mesopores and macropores (pore size > 50 nm).

In the present application, the term "all-silica molecular sieve", also known as pure silica molecular sieve or all-silica zeolite, has the usual meaning in the art. Specifically, the framework of such molecular sieve contains only Si and O elements, but no other elements, such as aluminum.

In the present application, the cavity structure in the molecular sieve crystal grains and its size and volume are obtained by transmission electron microscopy (TEM) testing, wherein the size of a single cavity structure refers to the maximum length of the line between two positions where the lines on the cavity wall of the cavity structure pass through the center of the cavity structure in the transmission electron microscope image of the molecular sieve, and the average value of the size of all the cavity structures in the crystal grain is calculated as the size of the cavity structure of the crystal grain, and the average value of the size of the cavity structure of 50 molecular sieve crystal grains is taken as the size of the cavity structure of the molecular sieve. For example, "the size of the cavity structure is within the range of 5-150nm" means that the size of the molecular sieve cavity structure represented by the above average value is within the range of 5-150nm; preferably, the length of the line between any two positions on the cavity wall of any cavity structure in each molecular sieve crystal grain passing through the center of the cavity structure is within the range of 5-150nm. The volume of the cavity structure is estimated according to the sphere, wherein the arithmetic mean of the sum of the lengths of the longest line and the shortest line among all lines between two positions on the cavity wall passing through the center of the cavity structure is taken as the spherical diameter, and then the spherical radius is obtained, and then the volume of the corresponding cavity structure is calculated according to the spherical volume formula. The percentage of the sum of the volumes of all the cavity structures in the molecular sieve crystal grains to the volume of the corresponding molecular sieve crystal grains is calculated, and the average value of the volume fractions of the cavity structures of 50 molecular sieve crystal grains is taken as the percentage of the cavity structure volume of the molecular sieve to the volume of the molecular sieve.

In the present application, the peak intensity of each characteristic peak in the $^{31}$P MAS NMR spectrum of the molecular sieve is represented by the integrated area of the corresponding characteristic peak.

In the present application, except for the contents clearly stated, any matters or items not mentioned are directly applicable to those known in the art without any changes. Moreover, any embodiment described herein can be freely combined with one or more other embodiments described herein, and the technical solutions or technical ideas formed thereby are deemed to be part of the original disclosure or original record of this application, and should not be regarded as new contents not disclosed or anticipated herein, unless a person skilled in the art considers that the combination is obviously unreasonable.

All patent and non-patent literatures, including but not limited to textbooks and journal articles, mentioned herein are incorporated herein by reference in their entirety.

After extensive research, the inventors of the present application discovered that when the hierarchical porous all-silica molecular sieve has both Brönsted acid and Lewis acid properties, when the molecular sieve is characterized by solid nuclear magnetic resonance technology using trimethylphosphine (TMP) as a probe molecule, in the resulting $^{31}$P MAS NMR spectrum, a characteristic peak characterizing the Brönsted acid property appears at a chemical shift of $-5\pm1$ ppm, a characteristic peak characterizing the Lewis acid property appears at a chemical shift of $-34\pm1$ ppm, and a characteristic peak characterizing the physical adsorption of TMP appears at a chemical shift of $-61\pm1$ ppm. Taking the characteristic peak of TMP physical adsorption as a comparison baseline, the characteristic peak characterizing the Brönsted acid property and the characteristic peak characterizing the Lewis acid property are respectively compared with the peak intensity of the characteristic peak of TMP physical adsorption. When the ratio of the peak intensity of each characteristic peak characterizing the two acid properties to the peak intensity of the characteristic peak of TMP physical adsorption is within a certain range, the hierarchical porous all-silica molecular sieve can have a high cyclohexanone oxime conversion rate and caprolactam selectivity, as well as a long reaction life in the cyclohexanone oxime gas-phase Beckmann reaction, thereby obtaining the present invention.

As described above, in a first aspect, the present application provides a hierarchical porous all-silica molecular sieve, which is characterized by solid nuclear magnetic resonance technology using trimethylphosphine (TMP) as a probe molecule, the $^{31}$P MAS NMR spectrum of the hierarchical porous all-silica molecular sieve shows three characteristic peaks at chemical shifts of $-5\pm1$ ppm, $-34\pm1$ ppm and $-61\pm1$ ppm, wherein the peak intensity of the characteristic peak at the chemical shift of $-5\pm1$ ppm is recorded as $N_1$, the peak intensity of the characteristic peak at the chemical shift of $-34\pm1$

ppm is recorded as $N_2$, and the peak intensity of the characteristic peak at the chemical shift of -61±1 ppm is recorded as $N_3$, then:

$X_1$ as defined by the following formula (1) is in the range of 0.10-0.30:

$$X_1 = N_1 / N_3 \text{ formula (1);}$$

and

$X_2$ as defined by the following formula (2) is in the range of 0.15-0.45:

$$X_2 = N_2 / N_3 \text{ formula (2).}$$

The hierarchical porous all-silica molecular sieve of the present application has abundant silanol group active centers and has both Brönsted acid and Lewis acid properties, and the value of $X_1$ is in the range of 0.10-0.30, and the value of $X_2$ is in the range of 0.15-0.45, so that the molecular sieve has suitable acidity and has a synergistic catalytic effect on the gas-phase Beckmann rearrangement reaction of cyclohexanone oxime, thereby significantly improving the caprolactam selectivity and extending the catalyst life.

In the present application, trimethylphosphine (TMP) is used as a probe molecule in the $^{31}P$ MAS NMR spectrum test of the hierarchical porous all-silica molecular sieve, and the test method is a conventional method in the art.

In a preferred embodiment, $X_1$ is in the range of 0.15-0.25; $X_2$ is in the range of 0.2-0.35. When $X_1$ and $X_2$ of the hierarchical porous all-silica molecular sieve are in the above preferred ranges, the hierarchical porous all-silica molecular sieve can obtain higher caprolactam selectivity and longer catalyst life.

In a preferred embodiment, the molecular sieve crystal has a plurality of cavity structures, wherein the size of the cavity structure is in the range of 5-150 nm, preferably in the range of 10-100 nm. In such preferred embodiments, the cavity structure is connected to the outside through micropores, and the cavity structure and the micropores connected thereto form a hierarchical porous structure.

In such preferred embodiments, the hierarchical porous all-silica molecular sieve of the present application has a plurality of relatively large-sized cavity structures inside, thereby having a large specific surface area and pore volume. At the same time, the cavity structure has abundant silanol group active centers, thereby providing a large number of independent reaction units, and the concentrated distribution of nested silanol groups (i.e., a number of silanol groups that are close to each other and interact with each other) makes the molecular sieve have both Brönsted acid and Lewis acid properties. In addition, the relatively large-sized cavity structure meets the needs of macromolecular reactions, and the reaction products are easier to flow out of the catalyst, avoiding the phenomenon of reduced catalytic activity of the catalyst due to pore blockage.

In a further preferred embodiment, all of the cavity structures account for 10-95 % of the total volume of the molecular sieve, and more preferably 15-90%. The shape of the cavity structure is not strictly limited, and can be, for example, one or more of a sphere, an ellipsoid, a cylinder, a polyhedron (such as a cube and an irregular cube), and an irregular shape, usually an irregular shape.

In a specific embodiment, the molecular sieve includes molecular sieve particles composed of a single crystal grain, and/or molecular sieve particles composed of a plurality of crystal grains aggregated. Optionally, the average particle size of the molecular sieve particles is 0.1-2 μ m, preferably 0.2-1.2 μm; the BET specific surface area is 300-650 $m^2/g$, preferably 350-550 $m^2/g$; the micropore specific surface area is 200-550 $m^2/g$, preferably 250-450 $m^2/g$; the total pore volume is 0.2-0.7 $cm^3/g$, preferably 0.3-0.5 $cm^3/g$; the mesopore volume is 0.1-0.5 $cm^3/g$, preferably 0.2-0.4 $cm^3/g$.

In a preferred embodiment, a hysteresis loop exists between the adsorption isotherm and the desorption isotherm of low temperature nitrogen adsorption of the molecular sieve. Further preferably, the initial relative pressure ($P/P_0$) at which the hysteresis loop appears is in the range of 0.2-0.99, preferably in the range of 0.4-0.99, and further preferably in the range of 0.4-0.6.

In a preferred embodiment, the configuration of the molecular sieve is selected from MFI topological structure (in which case the molecular sieve is Silicalite-1 molecular sieve), MEL topological structure (in which case the molecular sieve is Silicalite-2 molecular sieve), BEA topological structure, SVR topological structure, or a combination thereof.

In a second aspect, the present application provides a method for preparing a hierarchical porous all-silica molecular sieve, in particular the molecular sieve of the present application, comprising the following steps:

1) mixing a silicon source, a first templating agent, water, a silanizing agent and a structural filler to obtain a reaction mixture, wherein the structural filler is selected from an amphiphilic surfactant, a hard templating agent or a combination thereof;
2) sequentially performing a first hydrothermal crystallization treatment and a first calcination treatment on the

reaction mixture to obtain a molecular sieve intermediate;

3) mixing the molecular sieve intermediate, a second templating agent and water, and then sequentially performing a second hydrothermal crystallization treatment and a second calcination treatment.

In the method for preparing the hierarchical porous all-silica molecular sieve of the present application, by introducing the silanizing agent and the macromolecular structural filler into the molecular sieve synthesis raw materials, the layer stretching and pore expansion effect of the molecular sieve can be produced to prepare a molecular sieve material with open pores, and then the second templating agent is added for dissolution-recrystallization (i.e., the second hydrothermal crystallization) to prepare a hierarchical porous all-silica molecular sieve with special acid properties and an internal multi-cavity structure.

Specifically, in the method of the present application, in steps 1) and 2), the silanol groups of the silanizing agent and the silanol groups of the organosilicon source are hydrolyzed and condensed to produce stable Si-O-Si bonds, thereby ensuring the realization of the layer stretching and pore expansion effect. In addition, the long carbon chain of the silanizing agent and the structural filler of the amphiphilic surfactant can form a stable and controllable structural unit (the long carbon chain of the silanizing agent and the hydrophobic group of the surfactant are close to each other and interact with each other by van der Waals force), thereby playing a fine tuning role in the layer stretching and pore expansion; or using a size-controllable hard templating agent to play a space filling role, so that the molecular sieve produces an ordered, pore-controllable (controlled by the chain length of the alkyl chain of the silanizing agent) mesoporous structure; then in step 3), a second templating agent is introduced into the molecular sieve intermediate with open pores, and its dissolution and recrystallization mechanism is used to obtain the all-silica molecular sieve with specific acid properties and hierarchical porous structure of the present application. Specifically, in step 3), the inside of the crystal grains of the molecular sieve intermediate obtained in step 2) is dissolved in the presence of the second templating agent in a hydrothermal environment to produce a smaller cavity structure, and recrystallization occurs on the surface of the crystal grains, changing the crystal grain morphology; at the same time, due to the generation of this small cavity structure, the curvature of the hydroxyl distribution becomes larger, causing the hydroxyl density and orientation to change, increasing the hydrogen bonding effect, and leading to the generation of pseudo-B acid and L acid.

In a preferred embodiment, in step 1), the molar ratio of the silicon source: the first templating agent: water: the silanizing agent is 1: (0.01-2): (1-50): (0.02-0.2); and the weight ratio of silicon (in terms of $SiO_2$) to the structural filler in the reaction mixture is (5-40): 1.

In a further preferred embodiment, in step 1), the molar ratio of the silicon source: the first templating agent: water: the silanizing agent is 1: (0.02-0.3): (5-30): (0.04-0.15); the weight ratio of silicon (in terms of $SiO_2$) to the structural filler in the reaction mixture is (5-30): 1. According to this preferred embodiment, the obtained molecular sieve can further improve the caprolactam selectivity and catalyst life in the gas-phase Beckmann rearrangement reaction of cyclohexanone oxime.

According to the present application, the silicon source described in step 1) is not strictly limited, and can be various silicon sources suitable for the preparation of all-silica molecular sieves, preferably selected from organic silicate, solid silica gel, white carbon black, silica sol, or a combination thereof; more preferably selected from organic silicate, solid silica gel, white carbon black, or a combination thereof.

In a further preferred embodiment, the silicon source is selected from the organic silicate having a structure represented by the following formula (A), or a combination thereof:

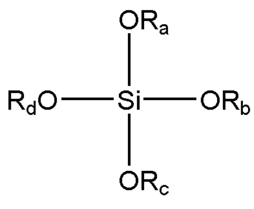

(A);

wherein $R_a$, $R_b$, $R_c$ and $R_d$ are each independently selected from linear or branched alkyl group having 1 to 6 carbon atoms, preferably each independently selected from linear alkyl group having 1 to 4 carbon atoms or branched alkyl group having 3 to 4 carbon atoms, and further preferably each independently selected from methyl, ethyl, n-propyl, isopropyl, n-butyl, sec-butyl, isobutyl or tert-butyl.

In a particularly preferred embodiment, the silicon source is selected from tetramethyl silicate, tetraethyl silicate, tetrabutyl silicate, dimethyl diethyl silicate, or a combination thereof.

According to the present application, the first templating agent in step 1) and the second templating agent in step 3) can be independently selected from various templating agents suitable for the preparation of all-silica molecular sieves, and the present application has no strict restrictions. In a preferred embodiment, the first templating agent and the second templating agent are both organic bases, preferably independently selected from quaternary ammonium bases, aliphatic

amines (such as trimethylamine, triethylamine and tripropylamine), aliphatic alcohol amines (such as ethanolamine), or a combination thereof.

In certain further preferred embodiments, the first templating agent and the second templating agent are each independently selected from a quaternary ammonium base having a structure represented by the following formula (B), or a combination thereof:

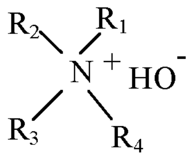

(B);

$R_1$, $R_2$, $R_3$ and $R_4$ are each independently selected from alkyl group having 1 to 4 carbon atoms, preferably each independently selected from linear alkyl group having 1 to 4 carbon atoms and branched alkyl group having 3 to 4 carbon atoms, and further preferably each independently selected from methyl, ethyl, n-propyl, isopropyl, n-butyl, sec-butyl, isobutyl and tert-butyl.

In a particularly preferred embodiment, the first templating agent and the second templating agent are each independently tetrapropylammonium hydroxide, or a mixture of tetrapropylammonium hydroxide and one or both of tetrapropylammonium chloride and tetrapropylammonium bromide.

According to the present application, the silanizing agent used in step 1) is not strictly limited, and silane agents with various elemental compositions and functional groups commonly used in the art can be used. In a preferred embodiment, the silanizing agent is selected from compounds with the general formula $R_5Si(R_6)(R_7)R_8$, or a combination thereof, wherein $R_5$, $R_6$, $R_7$ and $R_8$ are each independently halogen, alkyl, alkoxy, aryl, mercapto or amino, and at least one of them is alkyl, alkoxy, aryl, mercapto or amino; and the alkyl, alkoxy, mercapto and amino each independently have 1 to 18 carbon atoms.

In a further preferred embodiment, the silanizing agent is selected from dimethyldichlorosilane, N-phenyl-3-aminopropyltrimethoxysilane, phenyltrimethoxysilane, 1,7-dichlorooctylmethyltetrasiloxane, hexadecyltrimethoxysilane, octyltriethoxysilane, 3-aminopropyltrimethoxysilane, N-β-(aminoethyl)-γ-aminopropyltrimethoxysilane, 3-mercaptopropyltrimethoxysilane, or a combination thereof; further preferably selected from N-phenyl-3-aminopropyltrimethoxysilane, 3-aminopropyltrimethoxysilane, N-β-(aminoethyl)-γ-aminopropyltrimethoxysilane, 3-mercaptopropyltrimethoxysilane, or a combination thereof.

In certain preferred embodiments, the amphiphilic surfactant used in step 1) is a nonionic surfactant or a cationic surfactant, such as natural polysaccharides, hexadecyltrimethylammonium bromide, sodium dodecylbenzenesulfonate, etc.

According to the present application, the hard templating agent used in step 1) can be selected from various natural or synthetic polymer substances, such as cellulose, polysaccharide, starch, polyether, water-soluble polyamide, etc.

In a particularly preferred embodiment, the structural filler used in step 1) is selected from hexadecyltrimethylammonium bromide, sodium dodecylbenzenesulfonate, PEO-PPO-PEO block copolymer, mesoporous carbon, natural cellulose, or a combination thereof.

In certain preferred embodiments, step 1) comprises:

a) mixing the silicon source, the first templating agent and water to obtain a hydrolyzed sol of silicon;
b) adding the silanizing agent and the structural filler to the hydrolyzed sol of silicon respectively, and mixing them to obtain the reaction mixture.

In a further preferred embodiment, the mixing conditions in step a) include: stirring at 40-90 °C for 6-12 h; and the mixing conditions in step b) include: stirring at 20-50 °C for 2-4 h.

In a further preferred embodiment, the silicon source in step a) is an organic silicate, and step a) further comprises a hydrolysis and alcohol removal treatment after mixing the silicon source, the first templating agent and water to obtain the hydrolyzed sol of silicon; more preferably, the conditions of the hydrolysis and alcohol removal treatment include: stirring and hydrolyzing at 40-90 °C for 6-12 h; preferably stirring and hydrolyzing at 60-85 °C for 8-10 h. Further preferably, the hydrolysis and alcohol removal treatment is performed so that the mass content of the alcohol produced by hydrolysis of the organic silicate in the hydrolyzed sol of silicon is 10 ppm or less.

In a preferred embodiment, the conditions of the first hydrothermal crystallization treatment in step 2) include: the hydrothermal crystallization time is 0.25-7 days, the hydrothermal crystallization temperature is 130-200 °C; the pressure is autogenous pressure; the conditions of the first calcination treatment in step 2) include: the calcination temperature is

300-700 °C, and the calcination time is 1-16h.

In a preferred embodiment, the conditions of the first hydrothermal crystallization treatment in step 2) include: the hydrothermal crystallization time is 1-3 days, the hydrothermal crystallization temperature is 150-180 °C; the pressure is autogenous pressure; the conditions of the first calcination treatment in step 2) include: the calcination temperature is 400-600 °C, and the calcination time is 2-5h. The hierarchical porous all-silica molecular sieve prepared according to this embodiment has better catalytic activity.

In certain specific embodiments, in step 2), after the first hydrothermal crystallization treatment, the product of the first hydrothermal crystallization treatment is further subjected to a first filtration treatment, a first drying treatment, and then to the first calcination treatment, wherein the temperature of the first drying treatment is 50-120 °C and the time is 1-12h.

In a preferred embodiment, in step 3), the weight ratio of the molecular sieve intermediate, the second templating agent and water is 1:(0.01-1.5):(1-15); preferably 1:(0.1-1):(2-10). By adopting such a preferred embodiment, a molecular sieve with higher catalytic activity can be obtained.

In a preferred embodiment, the conditions of the second hydrothermal crystallization treatment in step 3) include: the hydrothermal crystallization time is 0.25-7 days, the hydrothermal crystallization temperature is 130-200 °C; the pressure is autogenous pressure; the conditions of the second calcination treatment in step 3) include: the calcination temperature is 300-700 °C, and the calcination time is 1-16h.

In a further preferred embodiment, the conditions of the second hydrothermal crystallization treatment in step 3) include: the hydrothermal crystallization time is 1-3 days, the hydrothermal crystallization temperature is 150-180 °C; the pressure is autogenous pressure; the conditions of the second calcination treatment in step 3) include: the calcination temperature is 400-600 °C, and the calcination time is 2-5h.

In certain specific embodiments, in step 3), after the second hydrothermal crystallization treatment, the product of the second hydrothermal crystallization treatment is further subjected to a second filtration treatment, a second drying treatment, and then to the second calcination treatment. The temperature of the second drying treatment is 50-120 °C and the time is 1-12h.

In a third aspect, a hierarchical porous all-silica molecular sieve prepared according to the method described in the second aspect of the present application is provided.

In a fourth aspect, a use of the hierarchical porous all-silica molecular sieve described in the first and third aspects of the present application in catalyzing the gas-phase Beckmann rearrangement reaction of cyclohexanone oxime is provided.

In a fifth aspect, a method for preparing caprolactam from cyclohexanone oxime is provided, comprising the step of subjecting cyclohexanone oxime to a gas-phase Beckmann rearrangement reaction in the presence of the hierarchical porous all-silica molecular sieve described in the first and third aspects of the present application.

In a preferred embodiment, the method comprises the step of contacting and reacting the raw material containing cyclohexanone oxime with the hierarchical porous all-silica molecular sieve in a nitrogen atmosphere. Preferably, the raw material comprises cyclohexanone oxime and a solvent, and the solvent is an alcohol, such as methanol, ethanol, butanol, cyclohexanol, etc.

In a further preferred embodiment, the conditions for the gas-phase Beckmann rearrangement reaction of cyclohexanone oxime include: a reaction temperature of 350-400 °C, a reaction pressure of 0-0.2 MPa, a molar ratio of nitrogen to cyclohexanone oxime of 0.1-30:1, cyclohexanone oxime accounts for 5-50% by weight of the total amount of cyclohexanone oxime and the solvent, and the weight space velocity of cyclohexanone oxime is 1-10 $h^{-1}$.

In certain specific embodiments, the present application provides the following technical solutions:

1. A hierarchical porous Silicalite-1 all-silica molecular sieve, wherein the hierarchical porous Silicalite-1 all-silica molecular sieve has the following $^{31}P$ MAS NMR characteristics:

   the peak intensity of the characteristic peak at the chemical shift of -5 ± 1 ppm of the molecular sieve is recorded as $N_1$; the peak intensity of the characteristic peak at the chemical shift of -34 ± 1 ppm of the molecular sieve is recorded as $N_2$; the peak intensity of the characteristic peak at the chemical shift of -61 ± 1 ppm of the molecular sieve is recorded as $N_3$;

   $X_1$ as defined by the following formula (1) is any value between 0.10 and 0.30;

$$X_1 = N_1 / N_3 \quad \text{formula (1);}$$

   and

   $X_2$ as defined by the following formula (2) is any value between 0.15 and 0.45;

$$X_2 = N_2 / N_3 \quad \text{formula (2).}$$

2. The hierarchical porous Silicalite-1 all-silica molecular sieve according to item 1, wherein $X_1$ is any value between 0.15 and 0.25; and $X_2$ is any value between 0.2 and 0.35.
3. The hierarchical porous Silicalite-1 all-silica molecular sieve according to item 1, wherein the molecular sieve crystal has multiple cavity structures; wherein the size of a single cavity structure is 5-150 nm, preferably 10-100 nm;

preferably, the volume of all the cavity structures accounts for 10-95% of the total volume of the molecular sieve, and further preferably 15-90%;
optionally, the shape of the cavity structure is selected from one or more of a sphere, a cube, an ellipsoid and an irregular cube.

4. The hierarchical porous Silicalite-1 all-silica molecular sieve according to item 1, wherein the molecular sieve comprises molecular sieve particles composed of a single crystal grain, and/or molecular sieve particles composed of a plurality of crystal grains aggregated;

optionally, the molecular sieve particles have an average particle size of 0.1-2 μm, preferably 0.2-1.2 μm; a BET specific surface area of 300-650 $m^2/g$, preferably 350-550 $m^2/g$; a micropore specific surface area of 200-550 $m^2/g$, preferably 250-450 $m^2/g$; a total pore volume of 0.2-0.7 $cm^3/g$, preferably 0.3-0.5 $cm^3/g$; a mesopore volume of 0.1- 0.5 $cm^3/g$, preferably 0.2- 0.4 $cm^3/g$;
optionally, a hysteresis loop exists between the adsorption isotherm and desorption isotherm of low-temperature nitrogen adsorption of the molecular sieve.

5. A method for preparing a hierarchical porous Silicalite-1 all-silica molecular sieve, comprising the following steps:

1) mixing a silicon source, a first templating agent, water, a silanizing agent and a structural filler to obtain a reaction mixture, wherein the structural filler is selected from an amphiphilic surfactant, a hard templating agent or a combination thereof;
2) sequentially performing a first hydrothermal crystallization treatment and a first calcination treatment on the reaction mixture to obtain a molecular sieve intermediate;
3) mixing the molecular sieve intermediate, a second templating agent and water, and then sequentially performing a second hydrothermal crystallization treatment and a second calcination treatment.

6. The method according to item 5, wherein in step 1), the molar ratio of the silicon source: the first templating agent: water: the silanizing agent is 1: (0.01-2): (1-50): (0.02-0.2); preferably 1: (0.02-0.3): (5-30): (0.04-0.15); the weight ratio of $SiO_2$ to the structural filler in the reaction mixture is (5-40) : 1, preferably (5-30) : 1.
7. The method according to item 5, wherein in step 1), the silicon source is selected from organic silicate, solid silica gel, white carbon black, silica sol, or a combination thereof; preferably selected from organic silicate, solid silica gel, white carbon black, or a combination thereof;
further preferably the organic silicate having a structure represented by the following formula (A):

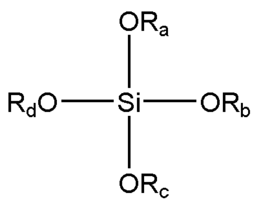
(A);

wherein $R_a$, $R_b$, $R_c$ and $R_d$ are each independently selected from linear or branched alkyl group having 1 to 6 carbon atoms, preferably $R_a$, $R_b$, $R_c$ and $R_d$ are each independently selected from linear alkyl group having 1 to 4 carbon atoms or branched alkyl group having 3 to 4 carbon atoms, and further preferably $R_a$, $R_b$, $R_c$ and $R_d$ are each independently selected from methyl, ethyl, n-propyl, isopropyl, n-butyl, sec-butyl, isobutyl or tert-butyl; particularly preferably, the organic silicate is selected from tetramethyl silicate, tetraethyl silicate, tetrabutyl silicate, dimethyl diethyl silicate, or a combination thereof.
8. The method according to item 5, wherein the first templating agent in step 1) and the second templating agent in step 3) are organic bases; and each is independently preferably at least one selected from quaternary ammonium bases,

aliphatic amines and aliphatic alcohol amines;
further preferably, the first templating agent and the second templating agent are each independently selected from a quaternary ammonium base having a structure represented by the following formula (B), or a combination thereof:

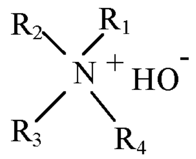 (B);

$R_1$, $R_2$, $R_3$ and $R_4$ are each independently selected from alkyl group having 1 to 4 carbon atoms, preferably each independently selected from linear alkyl group having 1 to 4 carbon atoms and branched alkyl group having 3 to 4 carbon atoms, and further preferably $R_1$, $R_2$, $R_3$ and $R_4$ are each independently selected from methyl, ethyl, n-propyl, isopropyl, n-butyl, sec-butyl, isobutyl and tert-butyl;

further preferably, the first templating agent and the second templating agent are each independently tetrapropylammonium hydroxide or a mixture of tetrapropylammonium hydroxide and one or both of tetrapropylammonium chloride and tetrapropylammonium bromide.

9. The method according to item 5, wherein in step 1), the silanizing agent is selected from compounds with the general formula $R_5Si(R_6)(R_7)R_8$, wherein $R_5$, $R_6$, $R_7$ and $R_8$ are each independently halogen, alkyl, alkoxy, aryl, mercapto or amino, and at least one of $R_5$, $R_6$, $R_7$ and $R_8$ is alkyl, alkoxy, aryl, mercapto or amino; and the alkyl, alkoxy, mercapto and amino each independently have 1 to 18 carbon atoms;
preferably, the silanizing agent is selected from dimethyldichlorosilane, N-phenyl-3-aminopropyltrimethoxysilane, phenyltrimethoxysilane, 1,7-dichlorooctylmethyltetrasiloxane, hexadecyltrimethoxysilane, octyltriethoxysilane, 3-aminopropyltrimethoxysilane, N-β-(aminoethyl)-γ-aminopropyltrimethoxysilane, 3-mercaptopropyltrimethoxysilane, or a combination thereof; further preferably selected from N-phenyl-3-aminopropyltrimethoxysilane, 3-aminopropyltrimethoxysilane, N-β-(aminoethyl)-γ-aminopropyltrimethoxysilane, 3-mercaptopropyltrimethoxysilane, or a combination thereof.
10. The method according to item 5, wherein in step 1), the structural filler is selected from hexadecyltrimethylammonium bromide, sodium dodecylbenzenesulfonate, PEO-PPO-PEO block copolymer, mesoporous carbon, natural cellulose, or a combination thereof.
11. The method according to item 5, wherein step 1) comprises:

a) mixing the silicon source, the first templating agent and water to obtain a hydrolyzed sol of silicon;
b) adding the silanizing agent and the structural filler to the hydrolyzed sol of silicon respectively, and mixing them to obtain the reaction mixture;

optionally, the mixing conditions in step a) include: stirring at 40-90 °C for 6-12 h;
optionally, the mixing conditions in step b) include: stirring at 20-50 °C for 2-4 h;
preferably, the silicon source is organic silicate, and step a) further comprises a hydrolysis and alcohol removal treatment after mixing the silicon source, the first templating agent and water to obtain the hydrolyzed sol of silicon;
optionally, the conditions for the hydrolysis and alcohol removal treatment include: stirring and hydrolyzing at 40-90 °C for 6-12 h; preferably stirring and hydrolyzing at 60-85 °C for 8-10 h.

12. The method according to item 5, wherein in step 3), the weight ratio of the molecular sieve intermediate: the second templating agent: water is 1: (0.01-1.5): (1-15); preferably 1: (0.1-1) : (2-10).
13. The method according to item 5, wherein the conditions of the first hydrothermal crystallization treatment in step 2) and the second hydrothermal crystallization treatment in step 3) independently include: the hydrothermal crystallization time is 0.25-7 days, the hydrothermal crystallization temperature is 130-200 °C; preferably, the hydrothermal crystallization time is 1-3 days, the hydrothermal crystallization temperature is 150-180 °C; the pressure is autogenous pressure;
the conditions of the first calcination treatment in step 2) and the second calcination treatment in step 3) independently include: a calcination temperature of 300-700 °C and a calcination time of 1-16h; preferably, a calcination temperature of 400-600 °C and a calcination time of 2-5h.
14. The hierarchical porous Silicalite-1 all-silica molecular sieve prepared according to the method described in any

one of items 5-13.

15. Use of the hierarchical porous Silicalite-1 all-silica molecular sieve described in any one of items 1-4 and 14 in catalyzing the gas-phase Beckmann rearrangement reaction of cyclohexanone oxime.

Examples

The present application is described in detail below through examples, but the present application is not limited to these examples.

In the following examples and comparative examples:
The $^{31}P$ MAS NMR spectrum of the samples were obtained by AVANCE III 600WB NMR spectrometer with trimethylphosphine (TMP) as a probe molecule, using 4 mm double resonance probe, Φ 4 mm $ZrO_2$ rotor, resonant frequency of 242.9 MHz, magic angle speed of 10 kHz, pulse width of 1.4 μs, cycle delay time of 1 s, and scan times of about 6000 times.

The X-ray diffraction (XRD) spectrum of the samples were measured on a Siemens D5005 X-ray diffractometer, the radiation source was Kα (Cu), and the test range 2θ was 0.5° - 70°.

The transmission electron microscopy (TEM) images of the samples were obtained by a Tecnai G2F20S-TWIN transmission electron microscope of FEI company. The cavity structure and size of the sample were obtained according to the TEM electron microscope test, and the volume fraction of the cavity structure volume to the molecular sieve volume was obtained by measuring the TEM images. The specific method is as follows: the volume of each cavity structure in the molecular sieve crystal grain is measured according to the TEM images (the volume of the cavity structure is estimated according to the sphere, wherein the arithmetic mean of the sum of the lengths of the longest line and the shortest line among all lines between two positions on the cavity wall passing through the center of the cavity structure is taken as the spherical diameter, and then the spherical radius is obtained, and then the volume of the corresponding cavity structure is calculated according to the spherical volume formula), the percentage of the sum of the volumes of all the cavity structures in the molecular sieve crystal grains to the volume of the corresponding molecular sieve crystal grains is calculated, and the average value of the volume fractions of the cavity structures of 50 molecular sieve crystal grains is taken as the percentage of the cavity structure volume of the molecular sieve to the volume of the molecular sieve.

The scanning electron microscope (SEM) images of the samples were obtained by a Hitachi S4800 high-resolution cold field emission scanning electron microscope. The average particle size of the samples was obtained by measuring the SEM images, wherein the particle sizes of 50 molecular sieve particles were measured and the arithmetic mean was taken as the average particle size of the molecular sieve.

The total specific surface area and total pore volume of the samples were measured using a ASAP245 static nitrogen adsorption instrument of Micromeritics company according to ASTM D4222-98 standard method.

The BET specific surface area, micropore specific surface area and mesopore volume of the samples were determined by low-temperature nitrogen adsorption-desorption method according to GB/T 19587-2004 standard and calculated using the BET equation.

The adsorption isotherm and desorption isotherm of low temperature nitrogen adsorption of the samples were measured according to ASTM D4222-98 standard method.

The infrared hydroxyl spectrum of the samples were measured by a Nicolet 8210 Fourier transform infrared spectrometer in the range of 400-4000 $cm^{-1}$.

In the following examples and comparative examples, unless otherwise specified, all reagents and raw materials used are commercially available products.

Example 1

1a) 104 g of tetraethyl silicate (0.5 mol), 65 g of 25 wt% tetrapropylammonium hydroxide (TPAOH, 0.08 mol) aqueous solution and 140 g of water (7.8 mol) were added to a 500 mL beaker in sequence, placed on a magnetic stirrer with heating and stirring functions to mix evenly, and stirred at 60 °C for 5 hours, and the evaporated water was replenished regularly to obtain a colorless and transparent silica gel solution;

1b) 9g of N-phenyl-3-aminopropyltrimethoxysilane (PHAPTMS, 0.035 mol) and 3 g of PEO-PPO-PEO triblock copolymer (P123, purchased from InnoChem, weight average molecular weight 5800) were added to the mixture of step 1a) and stirred for 2 hours;

2) The mixture obtained in step 1b) was transferred to a stainless steel closed reactor, and crystallized isothermally at 170 °C for 24 hours. The obtained product was filtered, washed, dried at 110 °C for 3 hours, and then calcined in a muffle furnace at 550 °C for 3 hours to obtain a molecular sieve intermediate S-1- T. The characterization results of its structural parameters are listed in Table 2. The values of $X_1$ and $X_2$ calculated by formula (1)-(2) are listed in Table 3;

3) 10g of the molecular sieve intermediate S-1- T, 20 g of 25 wt% tetrapropylammonium hydroxide (TPAOH, 5 g)

aqueous solution and 40 g of water were uniformly mixed (the weight ratio of the molecular sieve intermediate: the second templating agent (i.e., tetrapropylammonium hydroxide): water was 1:0.5:5.5), transferred to a stainless steel closed reactor, and crystallized isothermally at 170 °C for 24 h. The obtained product was filtered, washed, dried at 110 °C for 3 hours, and then calcined at 550 °C in a muffle furnace for 3 hours to obtain the all-silica molecular sieve S-1-M. The characterization results of its structural parameters are listed in Table 2.

The $^{31}$P-TMP MAS NMR spectrum of the molecular sieve S-1-M is shown in Figure 1, wherein there is a spectral peak corresponding to Brönsted acid at a chemical shift of -5 ± 1 ppm, and there is a spectral peak corresponding to Lewis acid at a chemical shift of -34 ± 1 ppm, indicating that the molecular sieve has both Brönsted acid and Lewis acid properties; wherein the peak intensity $N_1$ is 114450, $N_2$ is 158051, $N_3$ is 545002, and the calculated values of $X_1$ and $X_2$ are listed in Table 3.

The XRD spectrum of the molecular sieve S-1-M is shown in Figure 2, indicating that the molecular sieve has an MFI topological structure and is a Silicalite-1 molecular sieve.

The TEM electron microscope image of the molecular sieve S-1-M is shown in Figure 3. It can be seen that the crystal grains of the molecular sieve have hierarchical porous structures with multiple cavity structures inside, and the size of each cavity structure is in the range of 10-90nm.

The SEM electron microscope image of the molecular sieve S-1-M is shown in Figure 4. It can be seen that the particles of the molecular sieve have regular shapes and uniform sizes.

The low temperature nitrogen adsorption test result of the molecular sieve S-1-M is shown in Figure 5. It can be seen that there is an obvious hysteresis loop between the nitrogen adsorption isotherm and the desorption isotherm. The initial relative pressure ($P/P_0$) at which the hysteresis loop appears is 0.45.

The infrared hydroxyl spectrum of the molecular sieve S-1-M is shown in Figure 6. It can be seen that there are obvious silanol group active centers, among which the peak at 3740 $cm^{-1}$ is the terminal hydroxyl group, which acts as the side reaction center; the peak at 3690 $cm^{-1}$ is the ortho-hydroxyl group; and the peak at 3500 $cm^{-1}$ is the nested silanol group, which acts as the main reaction center.

The TEM image of the molecular sieve intermediate S-1-T that has not undergone the dissolution and recrystallization process (i.e., the second hydrothermal crystallization treatment) of step 3) is shown in Figure 7. Comparing Figure 7 with Figure 3, it can be seen that the molecular sieve intermediate S-1-T has created a large number of multi-cavity structures within the grains after the second hydrothermal crystallization treatment of step 3).

Comparative Example 1

The molecular sieve was prepared according to the method of Example 1, except that no silanizing agent and structural filler were added. The specific preparation conditions are listed in Table 1. The obtained molecular sieve was recorded as D-1, and the characterization results of its structural parameters are listed in Table 2, and the calculated values of $X_1$ and $X_2$ are listed in Table 3.

Comparative Example 2

The Silicalite-1 all-silica molecular sieve was prepared according to the method disclosed in patent application CN1338427 A, and the specific steps are as follows:

1) 104 g of tetraethyl silicate, 90 g of 22.5 wt% tetrapropylammonium hydroxide (TPAOH) aqueous solution and 110 g of water were added to a 500 mL beaker in sequence, placed on a magnetic stirrer with heating and stirring functions to mix evenly, and stirred at 75 °C for 5 hours, and the evaporated water was replenished regularly to obtain a colorless and transparent silica gel solution;
2) the above sol was transferred to a stainless steel closed reactor, crystallized isothermally at 170°C for 2 days, filtered, washed, dried at 120°C for 24 hours, and then calcined in a muffle furnace at 550°C for 5 hours;
3) 15 g of the calcined product and 55 g of 22.5 wt% tetrapropylammonium hydroxide (TPAOH) aqueous solution were mixed, crystallized isothermally at 170°C for 1 day, filtered, washed, dried at110°C for 12 hours, and then calcined in a muffle furnace at 550°C for 4 hours. The obtained molecular sieve is recorded as D-2, and the characterization results of its structural parameters are listed in Table 2, and the calculated values of $X_1$ and $X_2$ are listed in Table 3.

The $^{31}$P-TMP MAS NMR spectrum of the molecular sieve D-2 is shown in Figure 1. It can be seen that the molecular sieve D-2 has no obvious peaks corresponding to Brönsted acid or Lewis acid.

The TEM image of the molecular sieve D-2 is shown in Figure 10, and it can be seen that there is no cavity structure in the crystal grains of the molecular sieve D-2.

Comparative Example 3

The molecular sieve was prepared according to the method of Example 1, except that N-phenyl-3-aminopropyltrimethoxysilane was not used in step 1b). The obtained molecular sieve was denoted as D-3, and the characterization results of its structural parameters are listed in Table 2, and the calculated values of $X_1$ and $X_2$ are listed in Table 3.

Comparative Example 4

The molecular sieve was prepared according to the method of Example 1, except that the PEO-PPO-PEO triblock copolymer was not used in step 1b). The obtained molecular sieve was denoted as D-4, and the characterization results of its structural parameters are listed in Table 2, and the calculated values of $X_1$ and $X_2$ are listed in Table 3.

Examples 2-8

The molecular sieves were prepared according to the method of Example 1, except that the raw material ratios and the synthesis conditions were changed as shown in Table 1. The obtained molecular sieves were respectively recorded as S-2-M to S-8-M, and the characterization results of their structural parameters are listed in Table 2, and the calculated values of $X_1$ and $X_2$ are listed in Table 3.

Example 9

The molecular sieve was prepared according to the method of Example 1, except that the templating agent tetrapropylammonium hydroxide used in step 1a) and step 3) was replaced by tetrabutylammonium hydroxide (TBAOH), and the specific preparation conditions are listed in Table 1. The obtained molecular sieve was recorded as S-9-M, and the characterization results of its structural parameters are listed in Table 2, and the calculated values of $X_1$ and $X_2$ are listed in Table 3.

The XRD spectrum of the molecular sieve S-9-M is shown in Figure 8, indicating that the molecular sieve has a MEL topological structure and is a Silialite-2 molecular sieve.

Example 10

The molecular sieve was prepared according to the method of Example 1, except that the raw material ratios and the templating agent were changed as shown in Table 1, wherein the templating agent tetrapropylammonium hydroxide used in step 1a) and step 3) was replaced by tetraethylammonium hydroxide (TEAOH). The obtained molecular sieve was recorded as S-10-M, and the characterization results of its structural parameters are listed in Table 2, and the calculated values of $X_1$ and $X_2$ are listed in Table 3.

The XRD spectrum of the molecular sieve S-10-M is shown in Figure 9, indicating that the molecular sieve has a BEA topological structure.

Example 11

The molecular sieve was prepared according to the method of Example 1, except that:

the temperature of the first hydrothermal crystallization treatment of step 2) is 130 °C and the time is 4 days; the temperature of the first calcination treatment is 350 °C and the time is 8 hours; and

the temperature of the second hydrothermal crystallization treatment of step 3) is 130 °C and the time is 4 days; the temperature of the second calcination treatment is 350 °C and the time is 8 hours.

The obtained molecular sieve is recorded as S-11-M, and the characterization results of its structural parameters are listed in Table 2, and the calculated values of $X_1$ and $X_2$ are listed in Table 3.

Example 12

The molecular sieve was prepared according to the method of Example 1, except that the raw material ratios were changed as shown in Table 1. The obtained molecular sieve was recorded as S-12-M, and the characterization results of its structural parameters are listed in Table 2, and the calculated values of $X_1$ and $X_2$ are listed in Table 3.

Table 1 Operating conditions of the examples and comparative examples

| Molecular sieve number | Templating agent used (the first and second templating agents are the same) | First templating agent/silicon source (molar ratio) | Water/ silicon source (Molar Ratio) | Silanizing agent | Silanizing agent/silicon source (molar ratio) | Structural Filler | $SiO_2$ /structural filler (mass ratio) | Second templating agent of step 3)/molecular sieve (mass ratio) | Water of step 3)/molecular sieve (mass ratio) |
|---|---|---|---|---|---|---|---|---|---|
| S-1-M | TPAOH | 0.16 | 21 | PHAPTMS | 0.07 | P123 | 10 | 0.5 | 5.5 |
| S-2-M | TPAOH | 0.08 | 10 | TOMS | 0.07 | P123 | 10 | 0.5 | 10 |
| S-3-M | TPAOH | 0.16 | 10 | PHAPTMS | 0.10 | P123 | 30 | 1.0 | 5.5 |
| S-4-M | TPAOH | 0.10 | 21 | APTMS | 0.07 | CTAB | 10 | 0.5 | 3.3 |
| S-5-M | TPAOH | 0.16 | 10 | PHAPTMS | 0.03 | P123 | 20 | 0.3 | 5.5 |
| S-6-M | TPAOH | 0.16 | 5 | APTMS | 0.15 | CTAB | 10 | 0.5 | 7.5 |
| S-7-M | TPAOH | 0.15 | 10 | PHAPTMS | 0.07 | P123 | 5 | 1.0 | 5.5 |
| S-8-M | TPAOH | 0.16 | 30 | GCPMS | 0.04 | P123 | 10 | 1.0 | 5.5 |
| S-9-M | TBAOH | 0.16 | 21 | PHAPTMS | 0.07 | P123 | 10 | 0.5 | 5.5 |
| S-10-M | TEAOH | 0.16 | 21 | PHAPTMS | 0.07 | P123 | 10 | 0.5 | 5.5 |
| S-11-M | TPAOH | 0.16 | 21 | PHAPTMS | 0.07 | P123 | 10 | 0.5 | 5.5 |
| S-12-M | TPAOH | 0.50 | 35 | PHAPTMS | 0.20 | P123 | 35 | 1.5 | 15 |
| D-1 | TPAOH | 0.16 | 21 | - | - | - | - | 0.5 | 5.5 |
| D-2 | TPAOH | 0.20 | 10 | - | - | - | - | 0.83 | 2.84 |
| D-3 | TPAOH | 0.16 | 21 | - | - | P123 | 10 | 0.5 | 5.5 |
| D-4 | TPAOH | 0.16 | 21 | PHAPTMS | 0.07 | - | - | 0.5 | 5.5 |

In Table 1, TPAOH is tetrapropylammonium hydroxide, TBAOH is tetrabutylammonium hydroxide, TEAOH is tetraethylammonium hydroxide; PHAPTMS is N-phenyl-3-aminopropyltrimethoxysilane, APTMS is 3-aminopropyltriethoxysilane, GCPMS is 3-glycidoxypropyl(dimethoxy)methylsilane, TOMS is methyltrimethoxysilane; P123 is PEO-PPO-PEO triblock copolymer, and CTAB is hexadecyltrimethylammonium bromide.

In Table 1, the water in the calculation of "water/silicon source" also includes water from the first templating agent aqueous solution; the water in the calculation of "water/molecular sieve" also includes water from the second templating agent aqueous solution.

Table 2 Properties of the molecular sieves obtained in the examples and comparative examples

| Molecular sieve numbr | Average particle size of molecular sieve particles/ μm | BET specific surface area/ $m^2/g$ | Micropore specific surface area/ $m^2/g$ | Total pore volume / $cm^3/g$ | Micropore volume/ $cm^3/g$ | Mesopore volume/ $cm^3/g$ | Molecular sieve structure | Radial length of cavity structure/ nm | Percentage of cavity structure to the total volume of molecular sieve/% |
|---|---|---|---|---|---|---|---|---|---|
| S-1-M | 0.31 | 440 | 340 | 0.529 | 0.171 | 0.358 | MFI | 10-80 | 85 |
| S-2-M | 0.36 | 501 | 437 | 0.537 | 0.169 | 0.368 | MFI | 15-85 | 76 |
| S-3-M | 0.25 | 340 | 256 | 0.525 | 0.273 | 0.252 | MFI | 12-82 | 83 |
| S-4-M | 0.28 | 550 | 458 | 0.522 | 0.241 | 0.281 | MFI | 8-75 | 77 |
| S-5-M | 0.45 | 483 | 315 | 0.345 | 0.130 | 0.215 | MFI | 7-85 | 75 |

(continued)

| Molecular sieve numbr | Average particle size of molecular sieve particles/ μm | BET specific surface area/ $m^2/g$ | Micropore specific surface area/ $m^2/g$ | Total pore volume / $cm^3/g$ | Micropore volume/ $cm^3/g$ | Mesopore volume/ $cm^3/g$ | Molecular sieve structure | Radial length of cavity structure/ nm | Percentage of cavity structure to the total volume of molecular sieve/% |
|---|---|---|---|---|---|---|---|---|---|
| S-6-M | 0.39 | 530 | 448 | 0.362 | 0.150 | 0.212 | MFI | 12-86 | 82 |
| S-7-M | 0.28 | 590 | 369 | 0.395 | 0.142 | 0.253 | MFI | 14-80 | 83 |
| S-8-M | 0.43 | 470 | 456 | 0.345 | 0.111 | 0.234 | MFI | 13-86 | 80 |
| S-9-M | 0.36 | 476 | 387 | 0.513 | 0.253 | 0.260 | MEL | 10-78 | 81 |
| S-10-M | 0.35 | 610 | 457 | 0.369 | 0.159 | 0.210 | BEA | 15-78 | 77 |
| S-11-M | 0.28 | 390 | 274 | 0.458 | 0.174 | 0.287 | MFI | 13-82 | 78 |
| S-12-M | 0.36 | 462 | 324 | 0.478 | 0.232 | 0.246 | MFI | 20-74 | 72 |
| S-1-T | 0.35 | 659 | 489 | 0.583 | 0.327 | 0.256 | MFI | none | - |
| D-1 | 0.33 | 380 | 281 | 0.333 | 0.128 | 0.205 | MFI | none | - |
| D-2 | 0.36 | 400 | 352 | 0.363 | 0.116 | 0.247 | MFI | none | - |
| D-3 | 0.38 | 411 | 276 | 0.312 | 0.127 | 0.185 | MFI | none | - |
| D-4 | 0.36 | 698 | 376 | 0.544 | 0.278 | 0.266 | MFI | none | - |

Test Case

The molecular sieves prepared in the above examples and comparative examples were evaluated. The prepared molecular sieves were tableted and then crushed. The particles with a size of 20-60 mesh were used as catalysts for the gas-phase Beckmann rearrangement reaction of cyclohexanone oxime. The catalytic performance of the obtained molecular sieves was evaluated. The evaluation conditions were as follows:
the evaluation device is a normal pressure continuous flow fixed bed reactor with an inner diameter of 5 mm and a catalyst loading of 2 g. After loading the catalyst, it was pretreated in a nitrogen atmosphere at normal pressure and 350°C for 3 hours. The concentration of the raw material cyclohexanone oxime was 35% by weight, and the solvent was methanol. The reaction conditions included: the cyclohexanone oxime weight space velocity (WHSV, cyclohexanone oxime flow rate in the feed/catalyst weight in the reactor) was 2 $h^{-1,}$ the reaction temperature was 380°C, the nitrogen flow rate was 4 L/h, and the reaction time was 24 h and 120 h, respectively.

The reaction products were collected after cooling, and the concentrations of various substances were quantitatively analyzed using gas chromatography, using a 6890 gas chromatograph produced by Agilent, and an HP-5 column for analytical chromatography. The test conditions included: a vaporization chamber temperature of 250°C, a detection chamber temperature of 230°C, a column temperature of programmed temperature, a constant temperature of 110°C for 8 minutes, a temperature increase of 15°C/min to 230°C, and a constant temperature for 14 minutes; the results are listed in Table 3 below.
wherein:

cyclohexanone oxime conversion rate (mol%) = (molar content of cyclohexanone oxime in the feed - molar content of cyclohexanone oxime in the product)/molar content of cyclohexanone oxime in the feed × 100%;

caprolactam selectivity (mol%) = molar percentage of caprolactam in the product/(100-molar percentage of cyclohexanone oxime in the product) × 100%;

cyclohexanone oxime conversion rate reduction rate (%) = (cyclohexanone conversion rate for 24h- cyclohexanone conversion rate for 120h)/cyclohexanone conversion rate for 24h × 100%;

caprolactam selectivity reduction rate (%) = (caprolactam selectivity for 24h - caprolactam selectivity for 120h)/aprolactam selectivity for 24h × 100%.

Table 3 Test results of the molecular sieves obtained in examples and comparative examples

| Molecular sieve number | $X_1$ | $X_2$ | Reaction for 24h | | Reaction for 120 h | | Cyclohexanone oxime conversion rate reduction rate (%) | Caprolactam selectivity reduction rate (%) |
|---|---|---|---|---|---|---|---|---|
| | | | Cyclohexanone oxime conversion rate/mol% | Caprolactam selectivity/ mol% | Cyclohexanone oxime conversion rate/mol% | Caprolactam selectivity/mol% | | |
| S-1-M | 0.21 | 0.29 | 99.5 | 94.6 | 97.0 | 87.7 | 2.51 | 7.29 |
| S-2-M | 0.16 | 0.24 | 93.1 | 90.3 | 85.9 | 89.1 | 7.73 | 1.33 |
| S-3-M | 0.19 | 0.22 | 87.7 | 92.4 | 84.7 | 85.2 | 3.42 | 7.79 |
| S-4-M | 0.22 | 0.28 | 86.3 | 88.5 | 84.5 | 87.8 | 2.09 | 0.79 |
| S-5-M | 0.17 | 0.31 | 90.3 | 84.2 | 85.0 | 81.1 | 5.87 | 3.68 |
| S-6-M | 0.18 | 0.33 | 84.8 | 90.3 | 83.6 | 83.9 | 1.42 | 7.09 |
| S-7-M | 0.25 | 0.29 | 85.4 | 84.1 | 82.3 | 81.3 | 3.63 | 3.33 |
| S-8-M | 0.22 | 0.22 | 91.4 | 90.9 | 87.4 | 85.1 | 4.38 | 6.38 |
| S-9-M | 0.24 | 0.28 | 87.9 | 83.8 | 84.0 | 82.6 | 4.44 | 1.43 |
| S-10-M | 0.12 | 0.16 | 82.3 | 80.3 | 75.8 | 78.4 | 7.90 | 2.37 |
| S-11-M | 0.11 | 0.15 | 85.6 | 81.7 | 75.3 | 72.5 | 12.03 | 11.26 |
| S-12-M | 0.13 | 0.17 | 83.1 | 82.5 | 72.4 | 71.6 | 12.88 | 13.21 |
| S-1-T | 0.01 | 0.02 | 74.1 | 78.2 | deactivated | deactivated | - | - |
| D-1 | 0.04 | 0.01 | 76.3 | 74.0 | 60.7 | 62.6 | 20.45 | 15.41 |
| D-2 | 0.02 | 0.03 | 74.7 | 75.2 | 64.8 | 61.8 | 13.25 | 17.82 |
| D -3 | 0.02 | 0.03 | 73.1 | 71.8 | deactivated | deactivated | - | - |
| D -4 | 0.01 | 0.01 | 72.6 | 72.9 | deactivated | deactivated | - | - |

According to the data in Table 3, compared with the molecular sieve intermediate S-1-T and the molecular sieves obtained in Comparative Examples 1-4, the hierarchical porous all-silica molecular sieves S-1-M to S-12-M prepared in the examples of the present application have $X_1$ in the range of 0.1-0.3 and $X_2$ in the range of 0.15-0.45, and have higher catalytic activity in the gas-phase Beckmann rearrangement reaction of cyclohexanone oxime, higher cyclohexanone oxime conversion rate and caprolactam selectivity, and higher catalytic stability under long-term reaction conditions (120h).

By comparing S-1-M to S-9-M with S-10-M to S-12-M, it can be seen that $X_1$ of S-1-M to S-9-M is in the range of 0.15-0.25, and $X_2$ is in the range of 0.2-0.35, which make S-1-M to S-9-M have higher cyclohexanone oxime conversion rate and caprolactam selectivity, and higher catalytic stability under long-term reaction conditions (120h).

By comparing S-1-M with S-11-M, it can be seen that in Example 1 corresponding to the preferred embodiment of the present application, the obtained molecular sieve S-1-M has higher cyclohexanone oxime conversion rate and caprolactam selectivity, and higher catalytic stability under long-term reaction conditions (120h).

By comparing S-1-M with S-12-M, it can be seen that in Example 1, the molecular sieve is synthesized according to the molar ratio of the silicon source: the first templating agent: water: the silanizing agent of 1: (0.02-0.3): (5-30): (0.04-0.15), and the weight ratio of the molecular sieve intermediate: the second templating agent: water of 1: (0.1-1): (2-10)". The obtained molecule S-1-M has higher cyclohexanone oxime conversion rate and caprolactam selectivity, and

higher catalytic stability under long-term reaction conditions (120h).

The preferred embodiments of the present application are described in detail above; however, the present application is not limited to the specific details in the above embodiments. Within the technical concept of the present application, a variety of simple modifications can be made to the technical solution of the present application, and these simple modifications all fall within the protection scope of the present application.

It should also be noted that the various specific technical features described in the above specific embodiments can be combined in any suitable manner without contradiction. In order to avoid unnecessary repetition, the present application will not further describe various possible combinations.

In addition, the various embodiments of the present application may be arbitrarily combined, and as long as they do not violate the concept of the present application, they should also be regarded as the contents disclosed in the present application.

## Claims

1. A hierarchical porous all-silica molecular sieve, **characterized by** solid nuclear magnetic resonance technology using trimethylphosphine (TMP) as a probe molecule, the $^{31}P$ MAS NMR spectrum of the molecular sieve showing three characteristic peaks at chemical shifts of -5±1 ppm, - 34±1 ppm and -61±1 ppm, wherein the peak intensity of the characteristic peak at the chemical shift of -5±1 ppm is recorded as $N_1$, the peak intensity of the characteristic peak at the chemical shift of -34±1 ppm is recorded as $N_2$, and the peak intensity of the characteristic peak at the chemical shift of -61±1 ppm is recorded as $N_3$, then:

   $X_1$ as defined by the following formula (1) is in the range of 0.10-0.30, preferably in the range of 0. 15-0.25:

   $$X_1 = N_1 / N_3 \ (1);$$

   and
   $X_2$ as defined by the following formula (2) is in the range of 0.15-0.45, preferably in the range of 0.2-0.35:

   $$X_2 = N_2 / N_3 \ (2).$$

2. The molecular sieve according to claim 1, wherein the molecular sieve has a plurality of cavity structures in the crystal grains, and the size of the cavity structure is in the range of 5-150 nm, preferably in the range of 10-100 nm;

   preferably, the cavity structure volume of the molecular sieve accounts for 10-95% of the volume of the molecular sieve, and further preferably 15-90%;
   optionally, the shape of the cavity structure is selected from one or more of a sphere, an ellipsoid, a cylinder, a polyhedron and an irregular shape.

3. The molecular sieve according to any one of claims 1 to 2, wherein the molecular sieve comprises molecular sieve particles composed of a single crystal grain and/or molecular sieve particles composed of a plurality of crystal grains aggregated;
   optionally, the molecular sieve particles have an average particle size of 0.1-2 μm, preferably 0.2-1.2 μm; a BET specific surface area of 300-650 $m^2/g$, preferably 350-550 $m^2/g$; a micropore specific surface area of 200-550 $m^2/g$, preferably 250-450 $m^2/g$; a total pore volume of 0.2-0.7 $cm^3/g$, preferably 0.3-0.5 $cm^3/g$; and a mesopore volume of 0.1-0.5 $cm^3/g$, preferably 0.2-0.4 $cm^3/g$.

4. The molecular sieve according to any one of claims 1 to 3, wherein there is a hysteresis loop between the adsorption isotherm and the desorption isotherm of the low-temperature nitrogen adsorption test of the molecular sieve, preferably, the initial relative pressure ($P/P_0$) at which the hysteresis loop appears is in the range of 0.2-0.99, further preferably in the range of 0.4-0.99, and further more preferably in the range of 0.4-0.6.

5. A method for preparing the hierarchical porous all-silica molecular sieve according to any one of claims 1 to 4 comprising the following steps:

   1) mixing a silicon source, a first templating agent, water, a silanizing agent and a structural filler to obtain a

reaction mixture, wherein the structural filler is selected from an amphiphilic surfactant, a hard templating agent or a combination thereof, and the hard templating agent is selected from a natural or synthetic polymer material;
2) sequentially performing a first hydrothermal crystallization treatment and a first calcination treatment on the reaction mixture to obtain a molecular sieve intermediate;
3) mixing the molecular sieve intermediate, a second templating agent and water, and then sequentially performing a second hydrothermal crystallization treatment and a second calcination treatment,

preferably, the first templating agent and the second templating agent are organic bases, and are independently selected from quaternary ammonium bases, aliphatic amines, aliphatic alcohol amines, or a combination thereof.

**6.** The method according to claim 5, wherein in step 1):

the molar ratio of the silicon source: the first templating agent: water: the silanizing agent is 1: (0.01-2): (1-50): (0.02-0.2); preferably 1: (0.02-0.3): (5-30): (0.04-0.15); and
the weight ratio of silicon (calculated as $SiO_2$) to the structural filler in the reaction mixture is (5-40): 1, preferably (5-30) : 1.

**7.** The method according to claim 5 or 6, wherein in step 1), the silicon source is selected from organic silicate, solid silica gel, white carbon black, silica sol, or a combination thereof, preferably selected from organic silicate, solid silica gel, white carbon black, or a combination thereof;

further preferably, the silicon source is an organic silicate having a structure shown in the following formula (A):

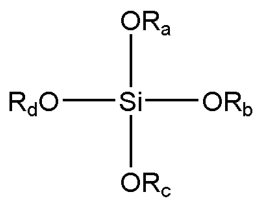
(A);

wherein $R_a$, $R_b$, $R_c$ and $R_d$ are each independently selected from linear or branched alkyl group having 1 to 6 carbon atoms, preferably each independently selected from linear alkyl group having 1 to 4 carbon atoms or branched alkyl group having 3 to 4 carbon atoms, and further preferably each independently selected from methyl, ethyl, n-propyl, isopropyl, n-butyl, sec-butyl, isobutyl or tert-butyl;
particularly preferably, the silicon source is selected from tetramethyl silicate, tetraethyl silicate, tetrabutyl silicate, dimethyl diethyl silicate, or a combination thereof.

**8.** The method according to any one of claims 5 to 7, wherein the first templating agent and the second templating agent are each independently selected from a quaternary ammonium base having a structure represented by the following formula (B), or a combination thereof:

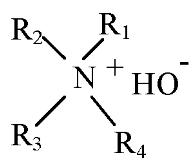
(B);

$R_1$, $R_2$, $R_3$ and $R_4$ are each independently selected from alkyl group having 1 to 4 carbon atoms, preferably each independently selected from linear alkyl group having 1 to 4 carbon atoms and branched alkyl group having 3 to 4 carbon atoms, and further preferably each independently selected from methyl, ethyl, n-propyl, isopropyl, n-butyl, sec-butyl, isobutyl and tert-butyl;
further preferably, the first templating agent and the second templating agent are each independently tetrapropylammonium hydroxide or a mixture of tetrapropylammonium hydroxide and one or both of tetrapropylammonium chloride and tetrapropylammonium bromide.

**9.** The method according to any one of claims 5 to 8, wherein in step 1), the silanizing agent is selected from compounds

with the general formula $R_5Si(R_6)(R_7)R_8$, or a combination thereof, wherein $R_5$, $R_6$, $R_7$ and $R_8$ are each independently halogen, alkyl, alkoxy, aryl, mercapto or amino, and at least one of them is alkyl, alkoxy, aryl, mercapto or amino; and the alkyl, alkoxy, mercapto and amino each independently have 1 to 18 carbon atoms;
preferably, the silanizing agent is selected from dimethyldichlorosilane, N-phenyl-3-aminopropyltrimethoxysilane, phenyltrimethoxysilane, 1,7-dichlorooctylmethyltetrasiloxane, hexadecyltrimethoxysilane, octyltriethoxysilane, 3-aminopropyltrimethoxysilane, N-β-(aminoethyl)-γ-aminopropyltrimethoxysilane, 3-mercaptopropyltrimethoxysilane, or a combination thereof; further preferably selected from N-phenyl-3-aminopropyltrimethoxysilane, 3-aminopropyltrimethoxysilane, N-β-(aminoethyl)-γ-aminopropyltrimethoxysilane, 3-mercaptopropyltrimethoxysilane, or a combination thereof.

**10.** The method according to any one of claims 5 to 9, wherein in step 1), the structural filler is selected from hexadecyltrimethylammonium bromide, sodium dodecylbenzenesulfonate, PEO-PPO-PEO block copolymer, mesoporous carbon, natural cellulose, or a combination thereof.

**11.** The method according to any one of claims 5 to 10, wherein step 1) comprises:

a) mixing the silicon source, the first templating agent and water to obtain a hydrolyzed sol of silicon; and
b) adding the silanizing agent and the structural filler to the hydrolyzed sol of silicon respectively, and mixing them to obtain the reaction mixture;
optionally, the mixing conditions in step a) include: stirring at 40-90 °C for 6-12 h;
optionally, the mixing conditions in step b) include: stirring at 20-50 °C for 2-4 h;
preferably, the silicon source is an organic silicate, and step a) further comprises a hydrolysis and alcohol removal treatment after mixing the silicon source, the first templating agent and water to obtain the hydrolyzed sol of silicon;
optionally, the conditions of the hydrolysis and alcohol removal treatment include: stirring and hydrolyzing at 40-90 °C for 6-12 h; preferably stirring and hydrolyzing at 60-85 °C for 8-10 h.

**12.** The method according to any one of claims 5 to 11, wherein in step 3), the weight ratio of the molecular sieve intermediate: the second templating agent: water is 1: (0.01-1.5): (1-15); preferably 1: (0.1-1): (2-10).

**13.** The method according to any one of claims 5 to 12, wherein the conditions of the first hydrothermal crystallization treatment in step 2) and the second hydrothermal crystallization treatment in step 3) each independently include: a hydrothermal crystallization time of 0.25-7 days, a hydrothermal crystallization temperature of 130-200°C; preferably, a hydrothermal crystallization time of 1-3 days, a hydrothermal crystallization temperature of 150-180°C; and a pressure of autogenous pressure; and
the conditions of the first calcination treatment in step 2) and the second calcination treatment in step 3) each independently include: a calcination temperature of 300-700°C and a calcination time of 1-16h; preferably, a calcination temperature of 400-600°C and a calcination time of 2-5h.

**14.** A method for preparing caprolactam from cyclohexanone oxime comprising a step of subjecting cyclohexanone oxime to a gas-phase Beckmann rearrangement reaction in the presence of the hierarchical porous all-silica molecular sieve according to any one of claims 1 to 4.

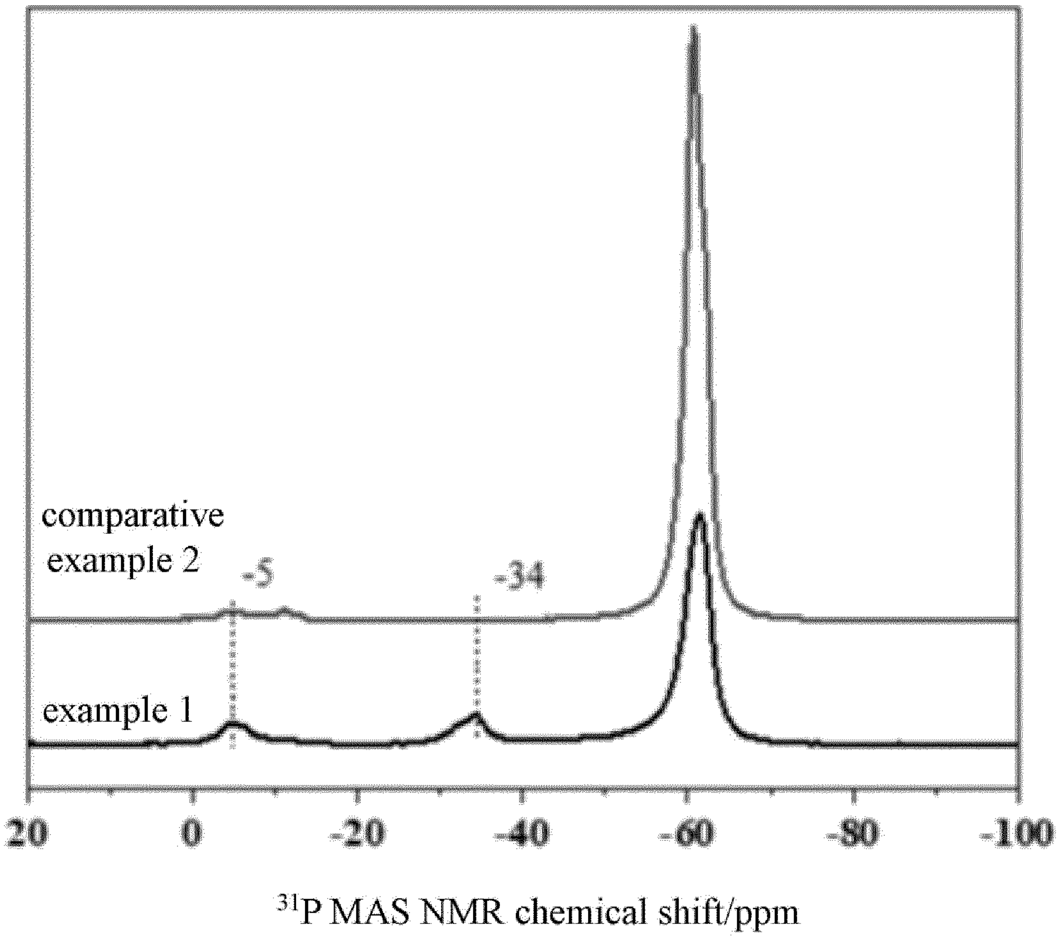
comparative
example 2
-5
-34
example 1
20
0
-20
-40
-60
-80
-100
31P MAS NMR chemical shift/ppm

FIG. 1

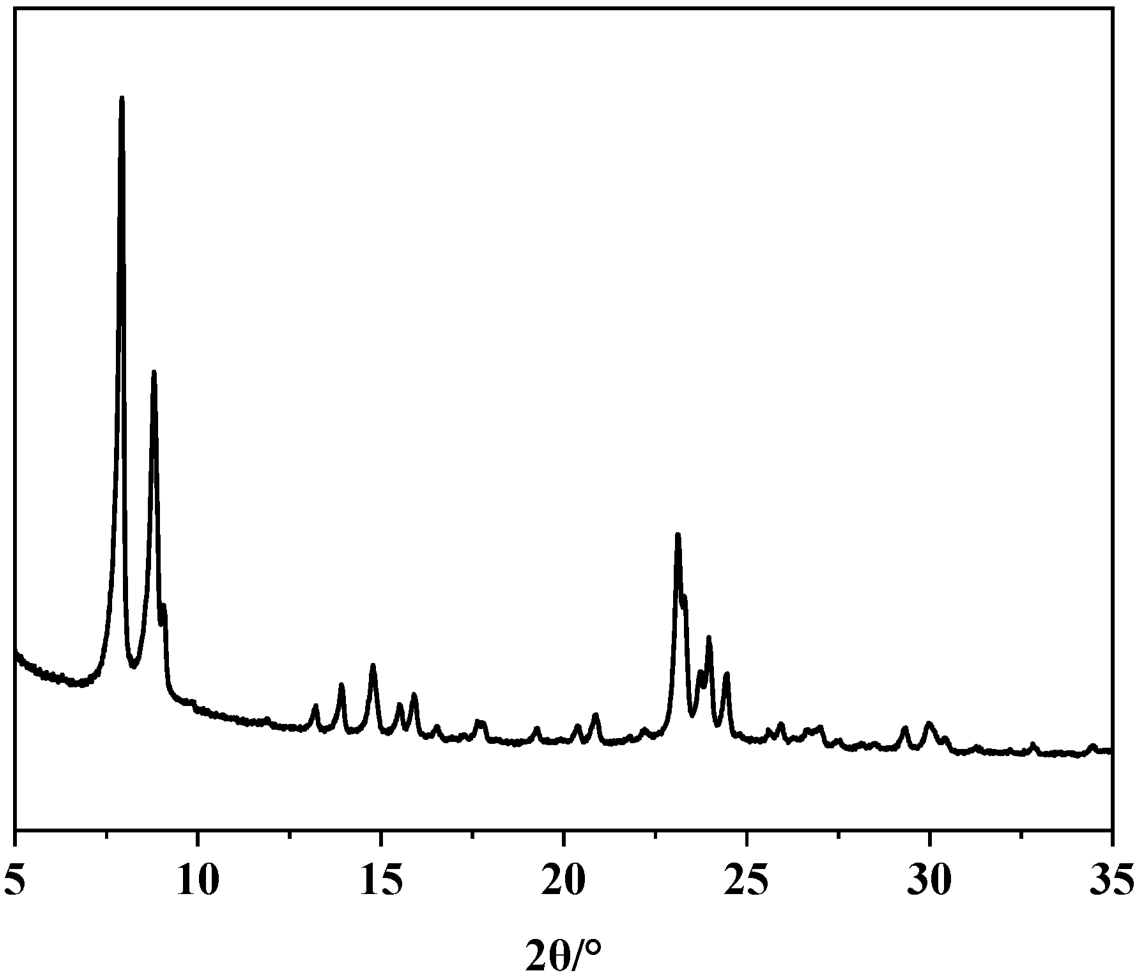


FIG. 2

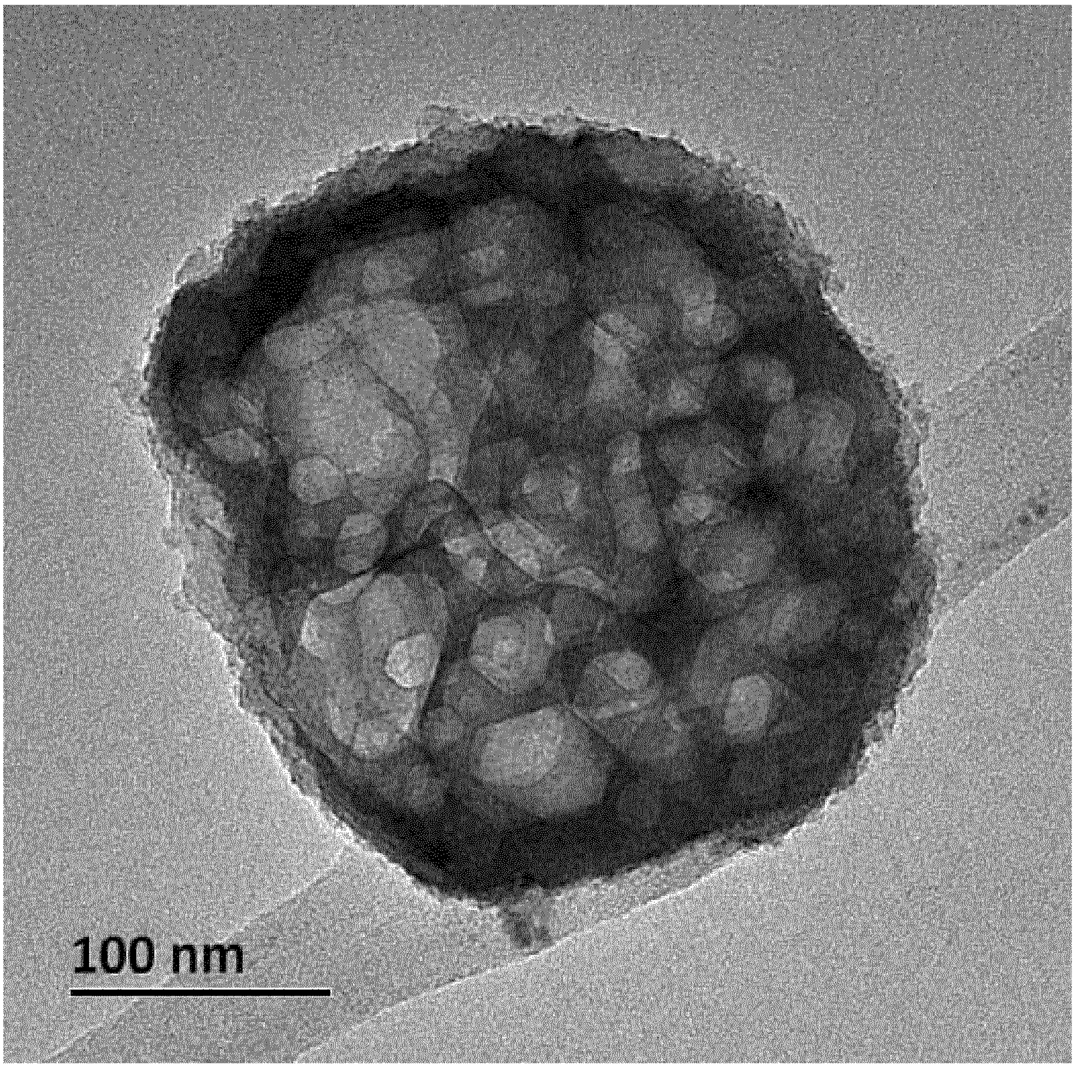


FIG. 3

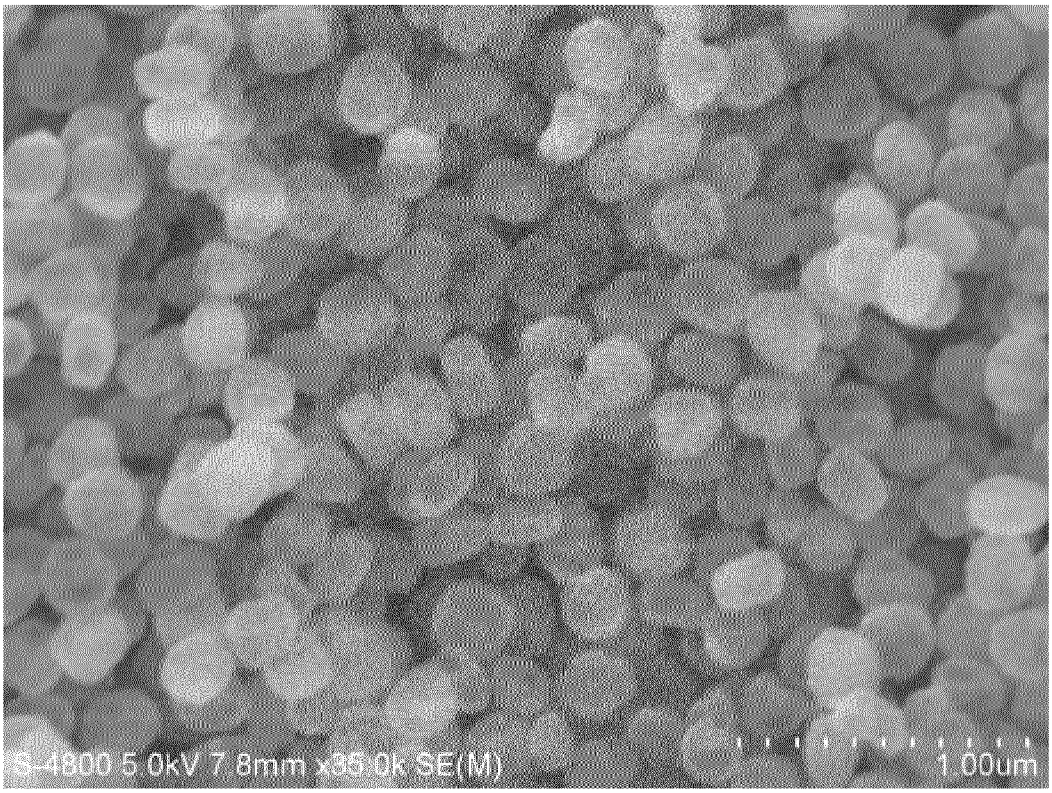
S-4800 5.0kV 7.8mm x35.0k SE(M)
1.00um

FIG. 4

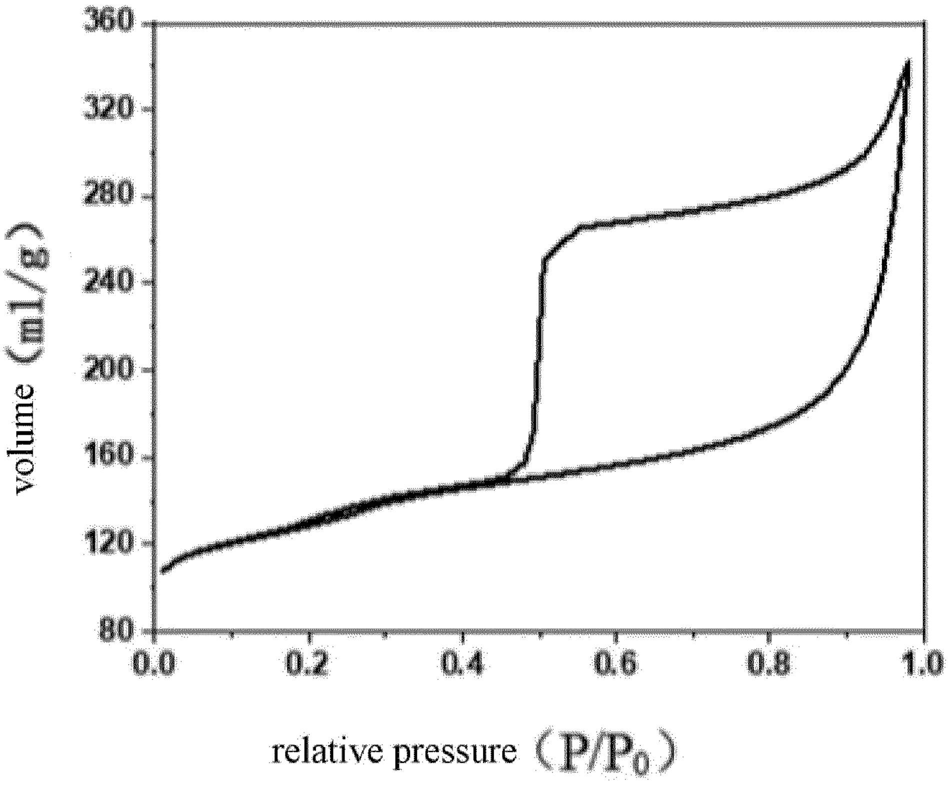
360
320
280
240
200
160
120
80
0.0
0.2
0.4
0.6
0.8
1.0
volume（ml/g）
relative pressure（P/P0）

FIG. 5

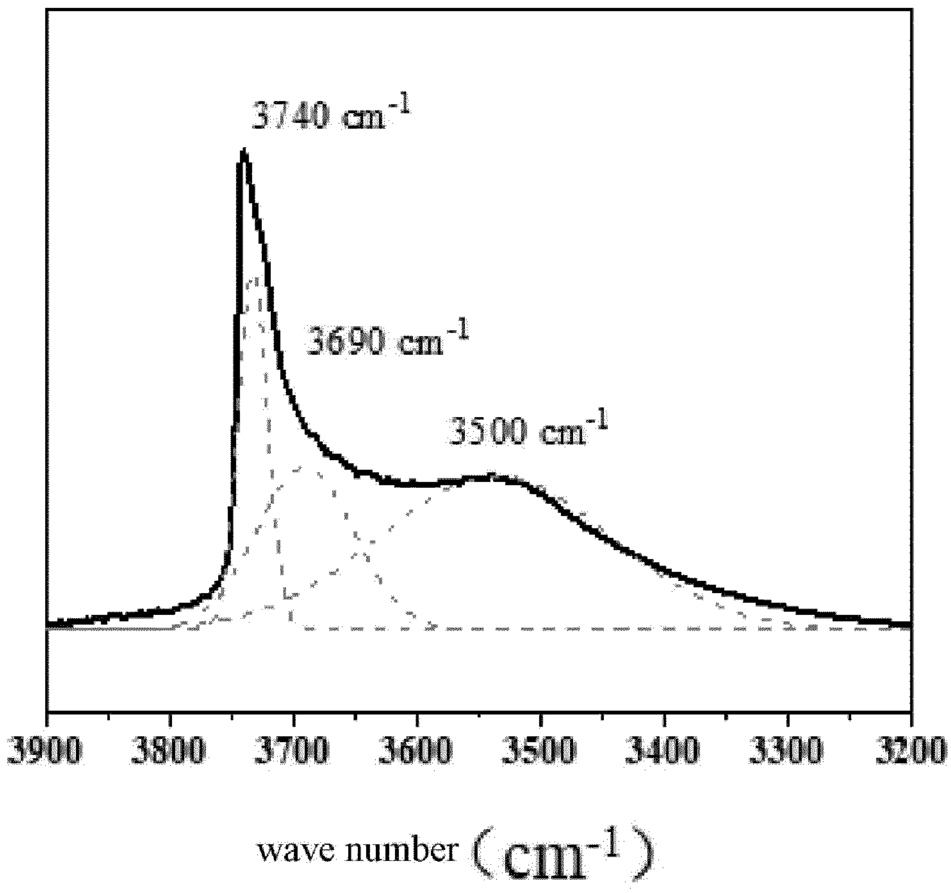
3740 cm-1
3690 cm-1
3500 cm-1
3900
3800
3700
3600
3500
3400
3300
3200
wave number (cm-1)

FIG. 6

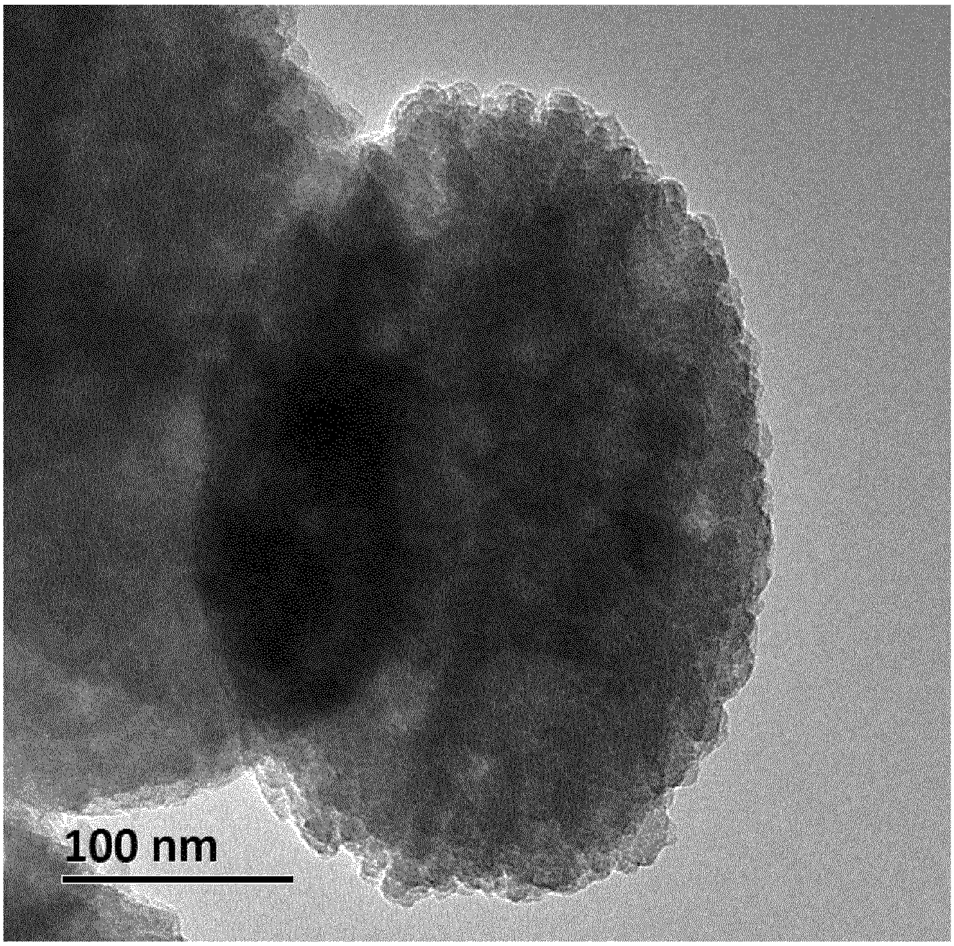


FIG. 7

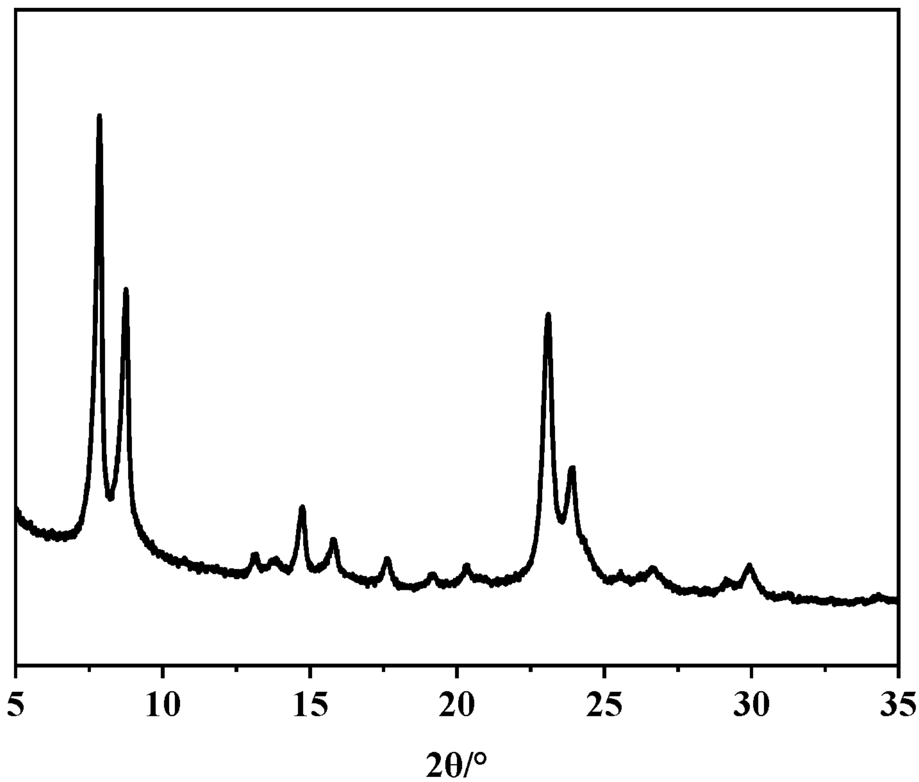


FIG. 8

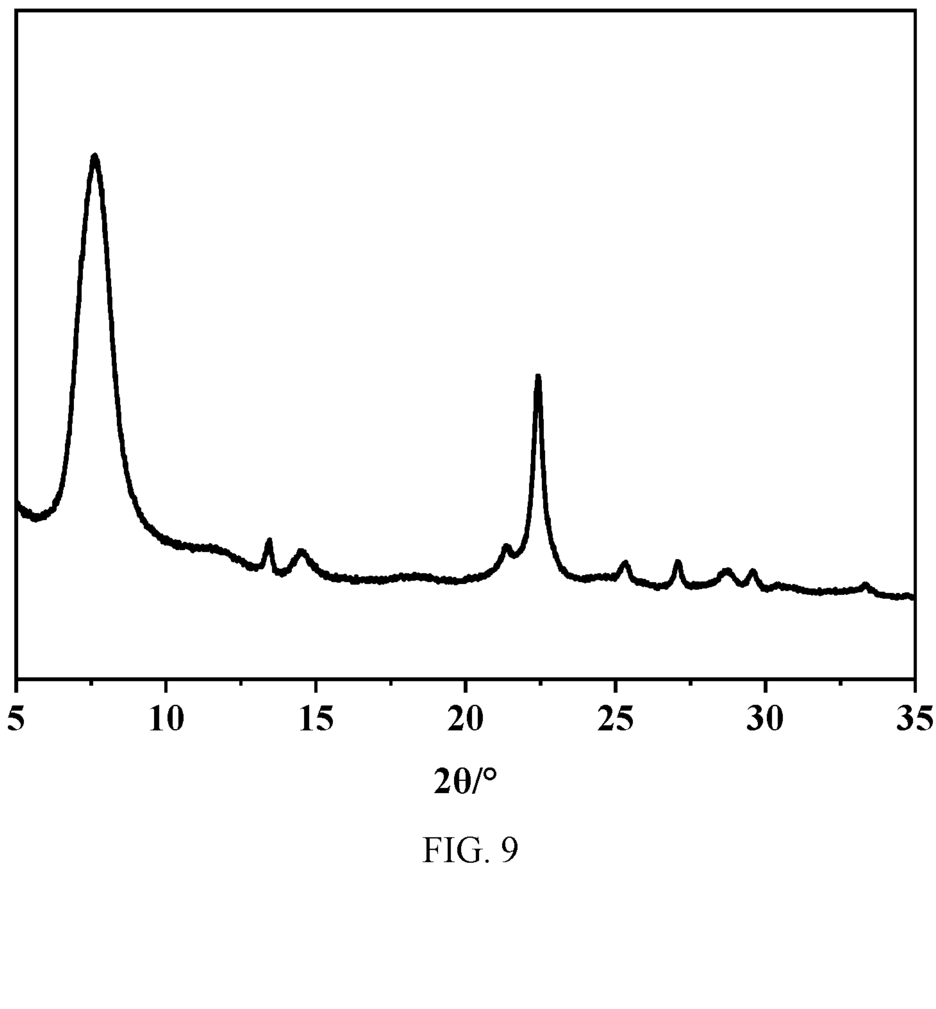


FIG. 9

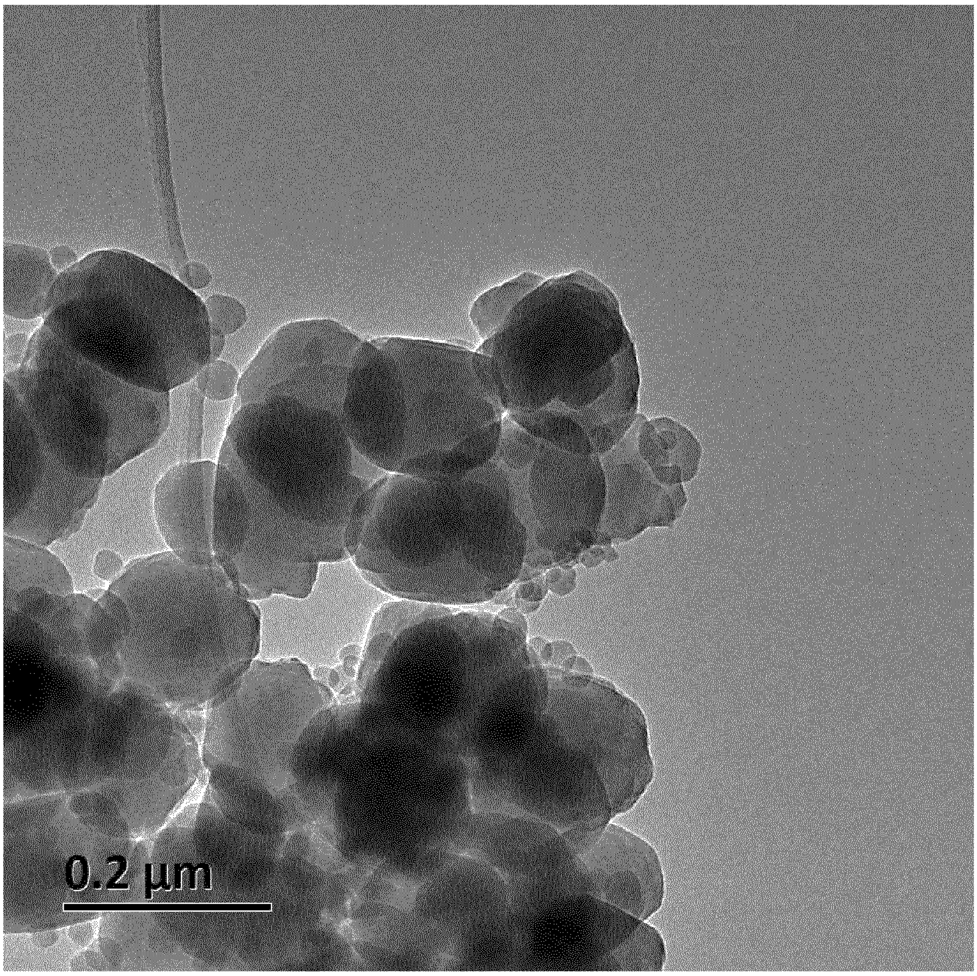


FIG. 10

**INTERNATIONAL SEARCH REPORT**

International application No. **PCT/CN2023/119697**

**A. CLASSIFICATION OF SUBJECT MATTER**

C01B 37/02(2006.01)i

According to International Patent Classification (IPC) or to both national classification and IPC

**B. FIELDS SEARCHED**

Minimum documentation searched (classification system followed by classification symbols)

IPC:C01B

Documentation searched other than minimum documentation to the extent that such documents are included in the fields searched

Electronic data base consulted during the international search (name of data base and, where practicable, search terms used)

CNABS, CNTXT, ENTXTC, VEN, WPABSC, WPABS, DWPI, CJFD, 超星读秀 CHAOXING DUXIU, CNKI, Web of Science: 中国石油化工, 中石化, 林民, 罗一斌, 彭欣欣, 朱斌, 舒兴田, 夏长久, 邢恩会, 张鹏, 张晓昕, 全硅, 多级, 分子筛, 沸石, Silicate, MFI, BEA, MEL, 胺, 模板, 共聚物, 硅, 硅烷, 硅氧烷, 核磁, NMR, ppm, 磺酸钠, 结构填充, 介孔碳, 纤维素, 溴化铵, 中间, molecular sieve, zeolite, template, mold+, silic+, +amine, silane, siloxane, intermediat+, P123, CTAB

**C. DOCUMENTS CONSIDERED TO BE RELEVANT**

| Category* | Citation of document, with indication, where appropriate, of the relevant passages | Relevant to claim No. |
|---|---|---|
| A | CN 113845124 A (DALIAN UNIVERSITY OF TECHNOLOGY) 28 December 2021 (2021-12-28)<br>claims 1-7 | 1-14 |
| A | US 2015119569 A1 (CHINA PETROLEUM & CHEMICAL CORP. et al.) 30 April 2015 (2015-04-30)<br>Claims | 1-14 |
| A | JP 2007261852 A (INSTITUTE OF RESEARCH AND INNOVATION) 11 October 2007 (2007-10-11)<br>entire document | 1-14 |
| A | ZASUKHIN, D. S. et al. "31P NMR Spectroscopy of Adsorbed Probe Molecules as a Tool for the Determination of the Acidity of Molecular Sieve Catalysts (A Review)"<br>*Petroleum Chemistry,* Vol. 61, No. 8, 30 July 2021 (2021-07-30), 875–894<br>ISSN: 0965-5441,<br>entire document | 1-14 |

☑ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **13 October 2023** | **09 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/119697**

**C. DOCUMENTS CONSIDERED TO BE RELEVANT**

| Category* | Citation of document, with indication, where appropriate, of the relevant passages | Relevant to claim No. |
|---|---|---|
| A | KOLODZIEJSKI, W. et al. "P-31 SOLID-STATE NMR-STUDY OF SPIN DIFFUSION IN THE MOLECULAR-SIEVE VPI-5 USING VARIABLE-SPEED MAS" *CHEMICAL PHYSICS LETTERS*, Vol. 191, No. 1, 2, 27 March 1992 (1992-03-27), 117-124 ISSN: 0009-2614, entire document | 1-14 |

Form PCT/ISA/210 (second sheet) (July 2022)

**INTERNATIONAL SEARCH REPORT**
**Information on patent family members**

International application No.
**PCT/CN2023/119697**

| Patent document cited in search report | | | Publication date (day/month/year) | Patent family member(s) | | | Publication date (day/month/year) |
|---|---|---|---|---|---|---|---|
| CN | 113845124 | A | 28 December 2021 | None | | | |
| US | 2015119569 | A1 | 30 April 2015 | KR | 20150050470 | A | 08 May 2015 |
| | | | | KR | 102307924 | B1 | 30 September 2021 |
| | | | | DE | 102014222018 | A1 | 30 April 2015 |
| | | | | US | 9656251 | B2 | 23 May 2017 |
| | | | | TW | 201515998 | A | 01 May 2015 |
| | | | | JP | 2015110510 | A | 18 June 2015 |
| | | | | JP | 6591740 | B2 | 16 October 2019 |
| | | | | MY | 168647 | A | 27 November 2018 |
| | | | | CN | 104556085 | A | 29 April 2015 |
| | | | | CN | 104556086 | A | 29 April 2015 |
| | | | | CN | 104556087 | A | 29 April 2015 |
| | | | | CN | 104556086 | B | 06 July 2016 |
| | | | | CN | 104556085 | B | 04 January 2017 |
| | | | | CN | 104556087 | B | 28 November 2017 |
| | | | | SG | 10201407071 | B | 04 January 2019 |
| | | | | IN | 400828 | B | 08 July 2022 |
| | | | | TW | 657047 | B1 | 21 April 2019 |
| | | | | SG | 10201407071 | A1 | 28 May 2015 |
| | | | | IN | 201405391 | I4 | 01 July 2016 |
| JP | 2007261852 | A | 11 October 2007 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1338427 A


### Non-patent literature cited in the description


- **LI W C** ; **LU A H** ; **PALKOVITS R et al.** Hierarchically structured monolithic silicalite-1 consisting of crystallized nanoparticles and its performance in the Beckmann rearrangement of cyclohexanone oxime [J. *Journal of the American Chemical Society*, 2005, vol. 127 (36), 12595-12600
- **GE, C.** ; **SUN, X. J.** ; **LIAN, D. D.** ; **LI, Z. K.** ; **WU, J. B.** Controllable synthesis and structure-performance relationship of silicalite-1 nanosheets in vapor phase beckmann rearrangement of cyclohexanone oxime. *Catal. Lett.*, 2020, vol. 151, 1488-1498